# NOUVEAU FASCICULE DE BREVET EUROPEEN

(11) **EP 1 800 658 B2**
(45) Date de publication et mention de la décision concernant l'opposition: **31.08.2016**
(45) Mention de la délivrance du brevet: 22.08.2012
(21) Numéro de dépôt: 06291979.0
(22) Date de dépôt: 19.12.2006
(51) Int. Cl.: A61K 8/81, A61K 8/898, A61K 8/34, A61Q 5/06

(54) **Composition cosmétique comprenant au moins un poly(vinyllactame) cationique, au moins un alcool gras et au moins une silicone aminée, procédé de traitement cosmétique et utilisation de la composition**
Kosmetische Zubereitung enthaltend mindestens ein kationisches Polyvinyllactam, minestens eine Fettalkohol und mindestens ein Aminosilikon, Verfahren zur kosmetischen Behandlung und Verwendung
Cosmetic composition comprising at least one cationic polyvinyllactam, at least one fatty alcohol and at least one aminosilicone, cosmetic treatment method and use

(30) Priorité: 22.12.2005 FR 0513194
(43) Date de publication de la demande: 27.06.2007
(73) Titulaire: L'Oréal, 75008 Paris (FR)
(72) Inventeur: Pasquet, Dorothée, 92270 Bois-Colombes (FR); Bebot, Cécile, 92110 Clichy (FR)
(74) Mandataire: Casalonga, Axel

(56) Documents cités:
- EP-A- 1 378 226
- EP-A- 1 426 038
- EP-A2- 0 312 992
- EP-A2- 1 319 389
- WO-A-00/68282
- WO-A-02/058646
- WO-A-02/058648
- WO-A1-2005/016291
- US-A- 5 077 040
- US-A1- 2001 031 270
- US-A1- 2003 147 827
- US-A1- 2004 115 156
- US-A1- 2004 133 994
- US-B1- 6 207 778
- Fiche produit "Dow Corning 929® Cationic Emulsion"
- Fiche produit "Wacker-Belsil® ADM 652, ADM 656, ADM 1100, ADM 1600, ADM 1650"
- Extrait de la banque de donées GNPD (Mintel); no d'accès 10239356 du 10-2005
- Extrait de la banque de donées CosIng sur le Polyquatemium-55
- Essais comapartifs en date du 16-10-2015

## Description

La présente invention concerne une composition cosmétique pour le traitement des fibres kératiniques, en particulier des fibres kératiniques humaines telles que les cheveux, comprenant au moins un polymère poly(vinyllactame) cationique, au moins un alcool gras et au moins une silicone aminée, une utilisation de cette composition pour le soin des cheveux ainsi qu'un procédé de traitement cosmétique la mettant en oeuvre.

Le document EP-A-1 378 226 décrit une composition cosmétique notamment de conditionnement des cheveux, comprenant, dans un milieu cosmétiquement acceptable, au moins une silicone à groupements ammonium quaternaire et au moins un alcool gras liquide. Les cheveux traités avec ces compositions sont brillants, ont un toucher doux et sans résidus.

Le document WO 02/058646 divulgue une composition de traitement des matières kératiniques, en particulier les cheveux, comprenant au moins un agent protecteur ou conditionneur et en outre au moins un polymère poly(alkyl) vinyllactame cationique. Ces associations permettent d'améliorer le dépôt de l'agent protecteur ou conditionneur des matières kératiniques ainsi que les propriétés cosmétiques.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et/ou les teintures. Il en résulte que les cheveux sont souvent difficiles à discipliner, en particulier ils sont difficiles à démêler ou à coiffer et les chevelures, même abondantes, conservent difficilement une coiffure de bon aspect en raison du fait que les cheveux manquent de vigueur.

Pour remédier à cela, il est maintenant usuel d'appliquer des produits de soin capillaires comprenant des agents de conditionnement qui facilitent le démêlage et le peignage des cheveux humides, assurant un bon maintien de la coiffure et leur communiquant, après séchage, de la douceur, du corps, du gonflant et de l'élasticité.

Ainsi ces produits de soin capillaires permettent généralement de discipliner les cheveux, de les lisser et de réduire le volume des cheveux frisottés, c'est-à-dire le volume de cheveux qui frisent de manière indésirable sous la forme de petites bouclettes, les rendant ainsi plus faciles à coiffer.

Ces compositions capillaires sont souvent formulées à partir d'alcools gras et comprennent généralement, en tant qu'agents de conditionnement des silicones cationiques.

Cependant bien que les silicones cationiques classiquement utilisées dans ces compositions capillaires confèrent généralement de bonnes propriétés cosmétiques, ces dernières ne permettent pas de discipliner suffisamment les cheveux frisottés ou de maîtriser leur volume de manière satisfaisante.

Il existe donc un réel besoin de trouver des compositions cosmétiques, notamment pour le soin ou pour le coiffage, qui ne présentent pas l'ensemble des inconvénients décrits ci-dessus tout en procurant aux cheveux des propriétés cosmétiques satisfaisantes, notamment en termes de douceur, de démêlage, d'aspect et de toucher.

De manière surprenante et avantageuse, la Demanderesse vient de découvrir qu'en associant un polymère poly(vinyllactame) cationique, un alcool gras et une silicone aminée, il était possible notamment d'obtenir des compositions cosmétiques qui permettent de discipliner, de lisser et de réduire le volume des cheveux frisottés tout en procurant aux cheveux de bonnes propriétés cosmétiques, en particulier en termes de douceur, de démêlage et de toucher.

La présente invention a donc notamment pour objet une composition cosmétique pour le traitement des fibres kératiniques, en particulier des fibres kératiniques humaines telles que les cheveux, comprenant une telle association.

Un autre objet de la présente invention consiste en une utilisation de la composition cosmétique selon l'invention pour le soin des cheveux.

L'invention a également pour objet un procédé de traitement cosmétique mettant en oeuvre la composition cosmétique selon l'invention.

L'invention a encore pour objet un dispositif aérosol comprenant la composition selon l'invention.

D'autres objets, caractéristiques, aspects et avantages de l'invention apparaîtront encore plus clairement à la lecture de la description et des exemples qui suivent.

Selon l'invention, la composition cosmétique pour le traitement des fibres kératiniques, en particulier des fibres kératiniques humaines telles que les cheveux, comprend, dans un milieu cosmétiquement acceptable,
- au moins un polymère poly(vinyllactame) cationique comprenant :
   - a) au moins un monomère de type vinyl lactame ou alkylvinyllactame;
   - b) au moins un monomère de structures (Ia) ou (Ib) suivantes : 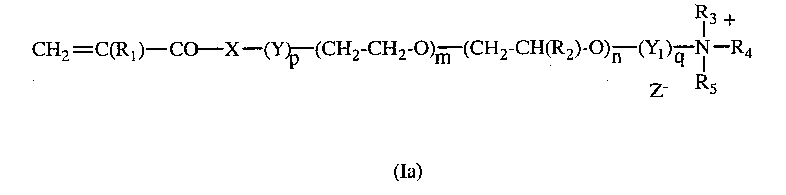 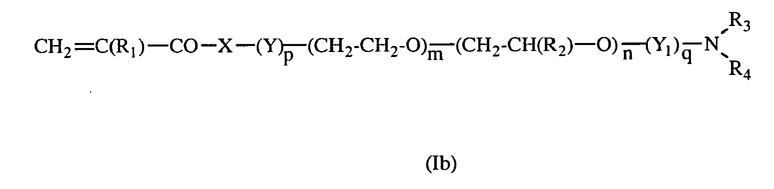 dans lesquelles :
      X désigne un atome d'oxygène ou un radical NR₆,
      R₁ et R₆ désignent, indépendamment l'un de l'autre, un atome d'hydrogène ou un radical alkyl linéaire ou ramifié en C₁-C₅,
      R₂ désigne un radical alkyle linéaire ou ramifié en C₁-C₄,
      R₃, R₄ et R₅ désignent, indépendamment l'un de l'autre, un atome d'hydrogène, un radical alkyle linéaire ou ramifié en C₁-C₃₀ ou un radical de formule (II) : 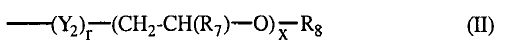
         Y, Y₁ et Y₂ désignent, indépendamment l'un de l'autre, un radical alkylène linéaire ou ramifié en C₂-C₁₆,
         R₇ désigne un atome d'hydrogène, ou un radical alkyle linéaire ou ramifié en C₁-C₄ ou un radical hydroxyalkyle linéaire ou ramifié en C₁-C₄,
         R₈ désigne un atome d'hydrogène ou un radical alkyle linéaire ou ramifié en C₁-C₃₀,
         p, q et r désignent, indépendamment l'un de l'autre, soit la valeur zéro, soit la valeur 1,
         m et n désignent, indépendamment l'un de l'autre, un nombre entier allant de 0 à 100,
         x désigne un nombre entier allant de 1 à 100,
         Z désigne un anion d'acide organique ou minéral,
         sous réserve que :
         - l'un au moins des substituants R₃, R₄, R₅ ou R₈ désigne un radical alkyle linéaire ou ramifié en C₉-C₃₀,
         - si m ou n est différent de zéro, alors q est égal à 1,
         - si m ou n sont égaux à zéro, alors p ou q est égal à 0 ;
- au moins un alcool gras présent dans une quantité variant de 0,1 à 30% en poids par rapport au poids total de la composition, et
- au moins une silicone aminée.

Les polymères poly(vinyllactames) cationiques utilisés dans la composition cosmétique selon l'invention comprennent :
- a) au moins un monomère de type vinyl lactame ou alkylvinyllactame;
- b) au moins un monomère de structures (la) ou (Ib) suivantes : 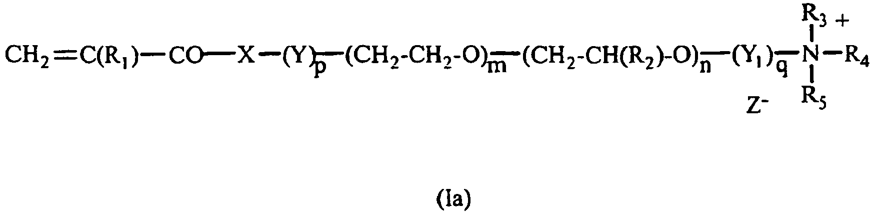 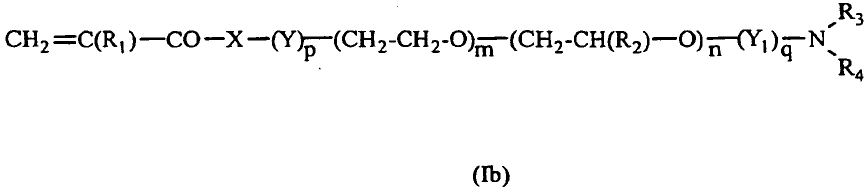 dans lesquelles :
   X désigne un atome d'oxygène ou un radical NR₆,
   R₁ et R₆ désignent, indépendamment l'un de l'autre, un atome d'hydrogène ou un radical alkyl linéaire ou ramifié en C₁-C₅,
   R₂ désigne un radical alkyle linéaire ou ramifié en C₁-C₄,
   R₃, R₄ et R₅ désignent, indépendamment l'un de l'autre, un atome d'hydrogène, un radical alkyle linéaire ou ramifié en C₁-C₃₀ ou un radical de formule (II) : 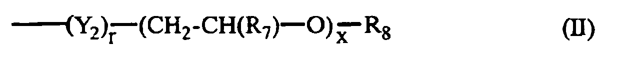
      Y, Y₁ et Y₂ désignent, indépendamment l'un de l'autre, un radical alkylène linéaire ou ramifié en C₂-C₁₆,
      R₇ désigne un atome d'hydrogène, ou un radical alkyle linéaire ou ramifié en C₁-C₄ ou un radical hydroxyalkyle linéaire ou ramifié en C₁-C₄,
      R₈ désigne un atome d'hydrogène ou un radical alkyle linéaire ou ramifié en C₁-C₃₀,
      p, q et r désignent, indépendamment l'un de l'autre, soit la valeur zéro, soit la valeur 1,
      m et n désignent, indépendamment l'un de l'autre, un nombre entier allant de 0 à 100,
      x désigne un nombre entier allant de 1 à 100,
      Z désigne un anion d'acide organique ou minéral,
      sous réserve que :
      - l'un au moins des substituants R₃, R₄, R₅ ou R₈ désigne un radical alkyle linéaire ou ramifié en C₉-C₃₀,
      - si m ou n est différent de zéro, alors q est égal à 1,
      - si m ou n sont égaux à zéro, alors p ou q est égal à 0 ;

Les polymères poly(vinyllactames) cationiques utilisés dans la composition cosmétique selon l'invention peuvent être réticulés ou non et peuvent aussi être des polymères blocs.

De préférence, le contre ion Z- des monomères de formule (la) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (la) pour laquelle, encore plus préférentiellement, m et n sont égaux à zéro.

Le monomère vinyl lactame ou alkylvinyllactame est de préférence un composé de structure (III) : 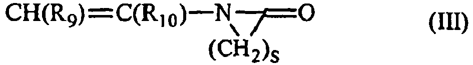 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (III) est la vinylpyrrolidone.

Les polymères poly(vinyllactame) cationiques utilisés dans la composition selon l'invention peuvent également contenir un ou plusieurs monomères supplémentaires, de préférence cationiques ou non ioniques.

A titre de composés utilisés de manière plus particulièrement préférée selon l'invention, on peut citer les terpolymères suivants comprenant au moins :
a)-un monomère de formule (III),
b)-un monomère de formule (la) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (Ib) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95% de monomère (a), 0,1 à 55% de monomère (c) et 0,25 à 50% de monomère (b).

De tels polymères sont décrits dans la demande de brevet WO-00/68282 dont le contenu fait partie intégrante de l'invention.

Comme polymères poly(vinyllactame) cationiques selon l'invention, on utilise notamment les terpolymères vinylpyrrolidone /diméthylaminopropylméthacrylamide / tosylate de dodécyldiméthylméthacrylamidopropylammonium, les terpolymères vinylpyrrolidone /diméthylaminopropylméthacrylamide/tosylate de cocoyldiméthylméthacrylamidopropylammonium, les terpolymères vinylpyrrolidone /diméthylaminopropylméthacrylamide / tosylate ou chlorure de lauryldiméthylméthacrylamidopropylammonium.

La masse moléculaire en poids des polymères poly(vinyllactame) cationiques utilisés dans la composition cosmétique selon la présente invention est de préférence comprise entre 500 et20 000 000. Elle est plus particulièrement comprise entre 200 000 et 2 000 000 et encore plus préférentiellement comprise entre 400 000 et 800 000.

Un polymère particulièrement préféré est le polymère commercialisé sous la dénomination «Styleze W20» par la société ISP qui est un terpolymère de vinylpyrrolidone / de diméthylaminopropylméthacrylamide et de chlorure de lauryldiméthylméthacrylamidopropylammonium.

Le(s) polymère(s) poly(vinyllactame) cationiques(s) est ou sont présents dans la composition cosmétique selon l'invention en une quantité variant de 0,05 à 30 % en poids, de préférence en une quantité variant de 0,1 à 15 % en poids et encore plus préférentiellement en une quantité variant de 0,2 à 10 % en poids, par rapport au poids total de la composition.

Par alcool gras, on entend au sens de la présente invention, tout alcool gras pur saturé ou insaturé, linéaire ou ramifié comportant au moins 8 atomes de carbone.

L'alcool gras n'est pas oxyalkyléné ou glycérolé.

L'alcool gras peut présenter la structure R-OH, dans laquelle R désigne un radical saturé ou insaturé, linéaire ou ramifié, comportant de 8 à 40 atomes de carbone et de préférence de 8 à 30 ; R désigne de préférence un groupement alkyle en C₁₂-C₂₄ ou alkényle en C₁₂-C₂₄. R peut être substitué par un ou plusieurs groupements hydroxy.

A titre d'exemple d'alcools gras, on peut citer les alcools laurique, cétylique, stéarylique, oléïque, béhénique, linoléique, undécylénique, palmitoléïque, arachidonique, érucique et leurs mélanges.

Le ou les alcools gras sont présents dans la composition dans une quantité variant de 0,1 à 30 %, de préférence en une quantité variant de 0,2 à 20 %, et encore plus préférentiellement dans une quantité variant de 0,5 à 10 % en poids, par rapport au poids total de la composition.

L'alcool gras peut représenter un mélange d'alcools gras, ce qui signifie que dans un produit commercial peuvent coexister plusieurs espèces d'alcools gras, sous forme d'un mélange.

A titre de mélange d'alcools gras, on peut citer l'alcool cétylstéarylique ou cétéarylique.

Avantageusement, l'alcool gras est solide ou pâteux à la température de 25°C. Par « alcool gras solide ou pâteux à 25°C », on entend au sens de la présente invention un alcool gras présentant une viscosité mesurée avec un rhéomètre avec un taux de cisaillement de 1s⁻¹ supérieure ou égale à 1 Pa.s.

De préférence, les alcools gras utilisés dans la composition cosmétique selon l'invention sont l'alcool cétylique et l'alcool cétéarylique.

Dans tout ce qui précède, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou par polycondensation de silanes convenablement fonctionnalisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles dont la partie alkyle est en C₁-C₁₀, les radicaux aryles et en particulier phényle.

Par silicone aminé, on entend au sens de la présente invention, toute silicone comportant au moins une fonction amine primaire, secondaire, tertiaire ou un groupement ammonium quaternaire.

Les silicones aminées utilisées dans la composition cosmétique selon la présente invention sont choisies parmi :
(a) les composés répondant à la formule (V) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (V)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -C_{q}H_{2q}L dans laquelle q est un nombre de 2 à 8 et L est un groupement aminé éventuellement quaternisé choisi parmi les groupements :
      - N(R2)-CH₂-CH₂-N(R²)₂ ;
      - N(R²)₂ ; -N⁺(R²)₃Q⁻ ;
      - N⁺(R²)(H)₂Q⁻;
      - N⁺(R²)₂HQ⁻ ;
      - N(R2)-CH₂-CH₂-N⁺(R²)(H)₂Q⁻,
   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.
   En particulier, les silicones aminées correspondant à la définition de la formule (V) sont choisies parmi les composés correspondant à la formule suivante : 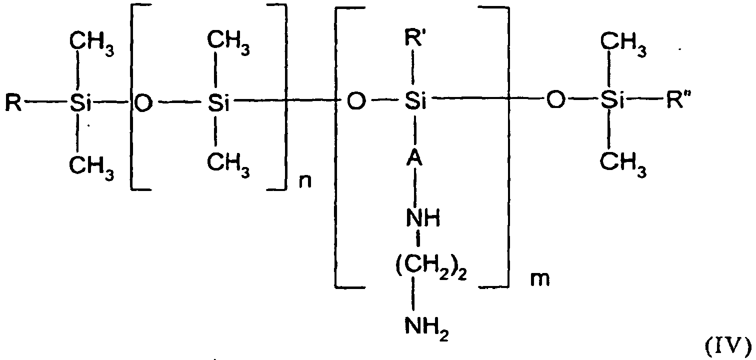 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.
   Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".
   Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.
   Dans cette catégorie de composés, on peut citer entre autres, le produit Belsil®ADM 652, commercialisée par Wacker.
   Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy/alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.
   Plus particulièrement, on peut citer le produit FluidWR® 1300, commercialisé par Wacker.
   Selon une quatrième possibilité, R, R" représentent un radical hydroxyle, R' représente un radical méthyle et A est un radical alkylène, en C₄-C₈, de préférence en C₄. Par ailleurs, m et n sont tels que la masse moléculaire moyenne en poids du composé est comprise entre 2000 et 10⁶. Plus particulièrement, n est compris entre 0 et 1999 et m est compris entre 1 et 2000, la somme de n et m étant comprise entre 1 et 2000.
   Un produit de ce type est notamment commercialisé sous la dénomination DC28299 par Dow Corning.
   Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).
   Un produit correspondant à la définition de la formule (V) est en particulier le polymère dénommé dans le dictionnaire CTFA "triméthylsilylamodiméthicone", répondant à la formule (VI) suivante : 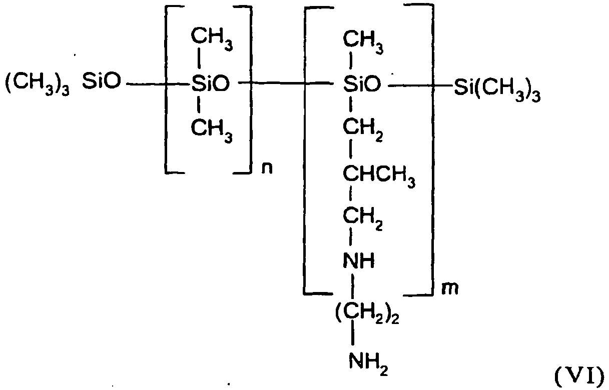 dans laquelle n et m ont les significations données ci-dessus conformément à la formule (V).
   De tels composés sont décrits par exemple dans EP 95238; un composé de formule (VI) est par exemple vendu sous la dénomination Q2-8220 par la société OSI.
(b) les composés répondant à la formule (VII) suivante : 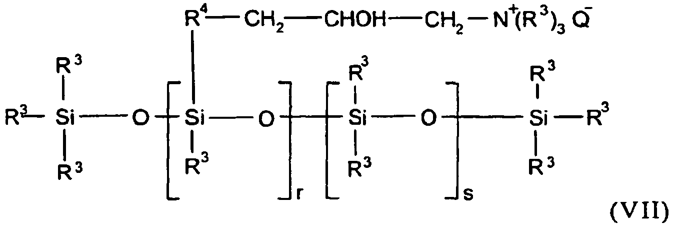 dans laquelle,
   R³ représente un radical hydrocarboné monovalent en C₁-C₁₈, et en particulier un radical alkyle en C₁-C₁₈, ou alcényle en C₂-C₁₈, par exemple méthyle ;
   R⁴ représente un radical hydrocarboné divalent, notamment un radical alkylène en C₁-C₁₈, ou un radical alkylèneoxy divalent en C₁-C₁₈, par exemple en C₁-C₈;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

   De tels composés sont décrits plus particulièrement dans le brevet US 4185087.
   Un composé entrant dans cette classe est celui vendu par la Société Union Carbide sous la dénomination "Ucar Silicone ALE 56".
c) les silicones ammonium quaternaire de formule (XII) : 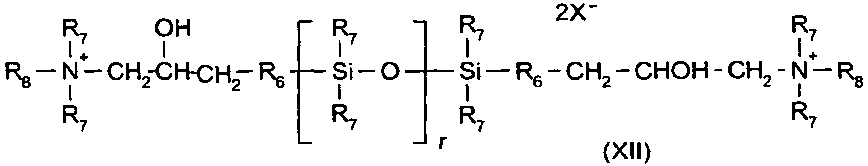 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alky-lèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇;
   X- est un anion tel qu'un ion halogénure, notamment chlorure ou un sel d'acide organique (acétate ...);
   r représente une valeur statistique moyenne de 2 à 200 et en particulier de 5 à 100 ;

   Ces silicones sont par exemple décrites dans la demande EP-A-0530974.
d) les silicones aminées de formule (XIII) : 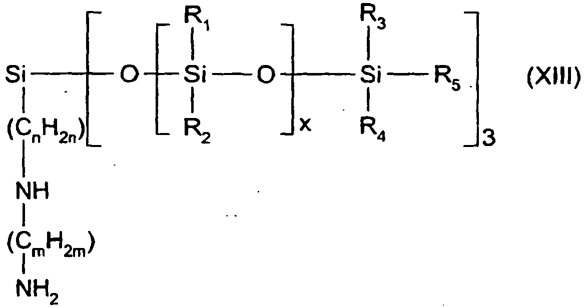 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
   et dans laquelle x est choisi de manière telle que l'indice d'amine soit compris entre 0,01 et 1 meq/g.

Les silicones particulièrement préférées conformément à l'invention sont les polysiloxanes à groupements aminés tels que les amodiméthicones ou les triméthylsilylamodiméthicones (CTFA 4ème édition 1997), et encore plus particulièrement les silicones à groupements ammonium quaternaire.

Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 929" par la Société Dow Corning, qui comprend, outre l'amodiméthicone, un agent de surface cationique comprenant un mélange de produits répondant à la formule : 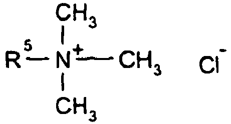 dans laquelle, R⁵ désigne des radicaux alcényle et/ou alcoyle en C₁₄-C₂₂ dérivés des acides gras du suif, et connu sous la dénomination CTFA "tallowtrimonium chloride",en association avec un agent de surface non ionique de formule :

C₉H₁₉-C₆H₄-(OC₂H₄)₁₀-OH,

connu sous la dénomination CTFA "Nonoxynol 10".

On peut également utiliser par exemple le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produitvendu sous la dénomination "Dow Corning Q2 7224" par la Société Dow Corning, comportant en association le triméthylsilylamodiméthicone de formule (C) décrite ci-dessus, un agent de surface non ionique de formule : C₈H₁₇-C₆H₄-(OCH₂CH2)₄₀-OH, connu sous la dénomination CTFA "octoxynol-40", un second agent de surface non ionique de formule: C₁₂H₂₅-(OCH₂-CH₂)₆OH, connu sous la dénomination CTFA "isolaureth-6", et du propylèneglycol.

La ou les silicones aminées peuvent être présentes dans la composition cosmétique dans une quantité variant de 0,01 à 20 % de préférence dans une quantité variant de 0,05 à 10 %, et encore plus préférentiellement dans une quantité variant de 0,1 à 5 % en poids, par rapport au poids total de la composition cosmétique.

De préférence, le rapport pondéral polyvinyllactame cationique de l'invention / alcool gras de l'invention est compris entre 0,1 et 5, le rapport pondéral polyvinyllactame cationique de l'invention / silicone aminée de l'invention est compris entre 0,1 et 5 et le rapport pondéral alcool gras / silicone aminée est compris entre 0,5 et 10.

La composition cosmétique peut comprendre en outre au moins une silicone oxyalkylénée ou une silicone choisie parmi les gommes de silicone.

Par silicone oxyalkylénée, on entend au sens de la présente invention, toute silicone comportant au moins un groupement oxyalkyléné de type (-CₓH₂ₓO-)ₐ dans lequel x peut varier de 2 à 6, et a est supérieur ou égal à 2.

Les silicones oxyalkylénées utilisables dans la composition cosmétique sont choisies parmi les formules générales (XIV), (XV), (XVI), (XVII) ou (XVIII) suivantes: 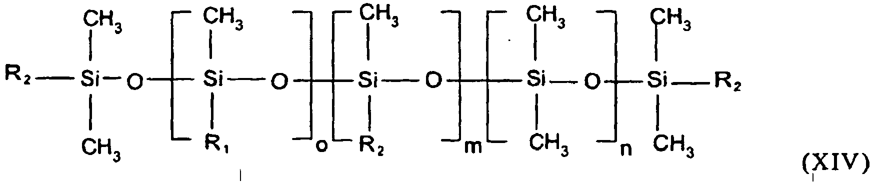 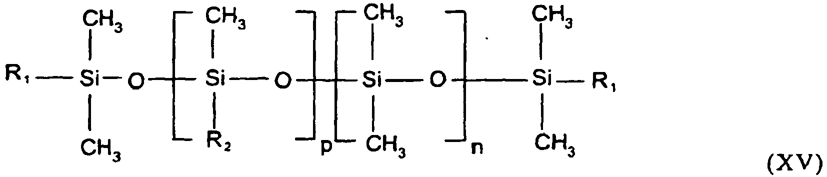 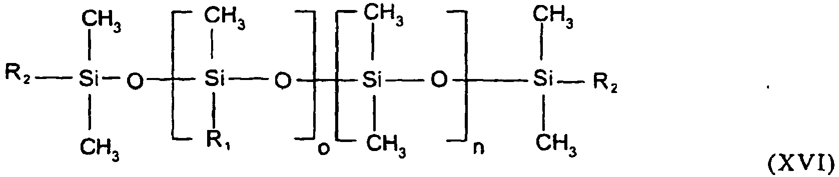 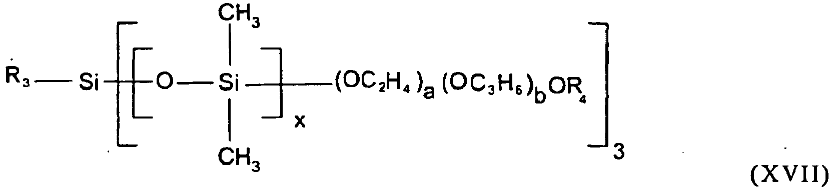 dans lesquelles:
- R₁, identique ou différent, représente un radical alkyle, linéaire ou ramifié, en C₁-C₃₀ ou phényle,
- R₂, identique ou différent, représente un radical -C_{c}H_{2c}-O-(C₂H₄O)ₐ(C₃H₆O)_{b}-R₅ ou un radical -C_{c}H_{2c}-O-(C₄H₈O)ₐ-R₅,
- R₃, R₄, identiques ou différents, désignent un radical alkyle, linéaire ou ramifié, en C₁ à C₁₂, et de préférence le radical méthyle,
- R₅, identique ou différent, est choisi parmi un atome d'hydrogène, un radical alkyle, linéaire ou ramifié, comprenant de 1 à 12 atomes de carbone, un radical alcoxy, linéaire ou ramifié, comprenant de 1 à 6 atomes de carbone, un radical acyle, linéaire ou ramifié, comprenant de 2 à 30 atomes de carbone, un radical hydroxyle, -SO₃M, aminoalcoxy en C₁-C₆ éventuellement substitué sur l'amine, aminoacyle en C₂-C₆ éventuellement substitué sur l'amine, -NHCH₂CH₂COOM, - N(CH₂CH₂COOM)₂, aminoalkyle éventuellement substitué sur l'amine et sur la chaîne alkyle, carboxyacyle en C₂-C₃₀, un groupement éventuellement substitué par un ou deux radicaux aminoalkyle substitués, -CO(CH₂)_{d}COOM, -COCHR₇(CH₂)_{d}COOM, - NHCO(CH₂)_{d}OH, -NH₃Y, un groupement phosphate,
- M, identique ou différent, désigne un atome d'hydrogène, Na, K, Li, NH₄ ou une amine organique,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 100,
- Y représente un anion minéral ou organique monovalenttel que halogénure (chlorure, bromure), sulfate, carboxylate (acétate, lactate, citrate),
sous réserve que lorsque la silicone est de formule (XV) avec R₅ désignant l'hydrogène alors n est supérieur à 12.

De telles silicones sont par exemple commercialisées par la société GOLDSCHMI DT sous les dénominations commerciales ABIL WE 09, ABIL EM 90, ABIL B8852, ABIL B8851, ABIL B 8843, ABIL B8842, par la société DOW CORNING sous les dénominations FLUID DC 190, DC 3225 C, Q2-5220, Q25354, Q2-5200, par la société RHODIA CHIMIE sous les dénominations SILBIONE HUILE 70646, RHODORSIL HUILE 10634, par la société GENERAL ELECTRIC sous les dénominations SF1066, SF1188, par la société SWS SILICONES sous la dénomination SILICONE COPOLYMER F 754, par la société AMERCHOL sous la dénomination SILSOFT BEAUTY AID SL, par la société SHIN-ETSU sous la dénomination KF 351, par la société WACKER sous la dénomination BELSIL DMC 6038, par la société SILTECH sous les dénominations SILWAX WD-C, SILWAX WD-B, SILWAX WD-IS, SILWAX WSL, SILWAX DCA 100, SILTECH AMINE 65, par la société FANNING CORPORATION sous les dénominations FANCORSIL SLA, FANCORSIL LIM1, par la société PHOENIX sous la dénomination PECOSIL.

Ces silicones sont notamment décrites dans les brevets US-A-5 070 171, US-A-5149765, US-A-5093452 et US-A-5091493.

De préférence, on utilise les silicones polyoxyalkylénées répondant aux formules générales (XV) ou (XVI). Plus particulièrement, ces formules répondent à au moins une des, et de préférence toutes les, conditions suivantes :
- c est égal à 2 ou 3.
- R₁ désigne le radical méthyle.
- R₅ représente un radical méthyle, un radical acyle en C₁₂-C₂₂, CO(CH₂)_{d}COOM.
- a varie de 2 à 25 et plus particulièrement de 2 à 15.
- b est égal à 0.
- n varie de 0 à 100.
- p varie de 1 à 20.

Les silicones polyoxyalkylénées peuvent également être choisies parmi les silicones de formule (XVIII) suivante :

([Z(R₂SiO)_{q}R'₂SiZO][(CₙH₂ₙO)ᵣ])ₛ (XVIII)

ladite formule (XVIII) dans laquelle:
- R₂ et R'₂, identiques ou différents, représentent un radical hydrocarboné monovalent en C₁-C₃₀,
- n est un nombre entier allant de 2 à 4,
- q est un nombre supérieur ou égal à 4, de préférence compris entre 4 et 200 et encore plus particulièrement entre 4 et 100.
- r est un nombre supérieur ou égal à 4, de préférence compris entre 4 et 200 et encore plus particulièrement entre 5 et 100.
- s est un nombre supérieur ou égal à 4, de préférence compris entre 4 et 1 000 et encore plus particulièrement entre 5 et 300.
- Z représente un groupe organique divalent qui est lié à l'atome de silicium adjacent par une liaison carbone-silicium et au bloc polyoxyalkylène (CₙH₂ₙO) par un atome d'oxygène,
- le poids moléculaire moyen de chaque bloc siloxane est compris entre environ 400 et environ 10 000, celui de chaque bloc polyoxyalkylène étant compris entre environ 300 et environ 10 000,
- les blocs siloxane représentent de 10% environ à 95% environ en poids du copolymère bloc,
- le poids moléculaire moyen en nombre du copolymère bloc pouvant aller de 2 500 à 1 000 000 et de préférence compris entre 3 000 et 200 000 et encore plus particulièrement entre 6 000 et 100 000.
R₂ et R'₂ sont préférentiellement choisis parmi le groupe comprenant les radicaux alkyles linéaires ou ramifiés comme par exemple les radicaux méthyle, éthyle, propyle, butyle, pentyle, hexyle, octyle, décyle, dodécyle, les radicaux aryles comme par exemple phényle, naphtyle, les radicaux aralkyls ou alkylaryles comme par exemple benzyle, phényléthyle, les radicaux tolyle, xylyle.
Z est de préférence -R"-, -R"-CO-, -R"-NHCO-, -R"-NH-CO-NH-R"'-, -R'-OCONH-R"'-NHCO-, où R" est un groupe alkylène divalent, linéaire ou ramifié en C₁-C₆, comme par exemple l'éthylène, le propylène ou le butylène, linéaire ou ramifié et R"' est un groupe alkylène divaient ou un groupe arylène divalent comme -C₆H₄-, -C₆H₄-C₆H₄-, -C₆H₄-CH₂-C₆H₄-, -C₆H₄-C(CH₃)₂C₆H₄.

Encore plus préférentiellement, Z représente un radical alkylène divalent, plus particulièrement le radical -C₃H₆-ou le radical C₄H₈, linéaires ou ramifiés.

La préparation des copolymères blocs est décrite dans la demande européenne EP 0 492 657 A1, dont l'enseignement est inclus dans la présente description.

De tels produits sont par exemple commercialisés sous la dénomination SILICONE FLUID FZ-2172 par la société OSI.

Les silicones utilisées dans les compositions cosmétiques peuvent se présenter sous forme de solutions aqueuses ou éventuellement sous forme de dispersions ou d'émulsions aqueuses.

De manière préférée, la ou les silicones utilisables dans la composition cosmétique peuvent être aussi des gommes de silicone.

Les gommes de silicone utilisables dans la composition cosmétique selon l'invention sont notamment des polydiorganosiloxanes ayant des poids moléculaires moyens en poids élevés compris entre 200 000 et 1 000 000 utilisés seuls ou en mélange dans un solvant. Ce solvant peut être choisi parmi les silicones volatiles, les huiles polydiméthyl-siloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables sont des mélanges tels que :
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

La composition cosmétique peut comprendre en outre un ou plusieurs polymères fixants.

Par polymère fixant, on entend au sens de la présente invention tout polymère permettant de conférer une forme ou de maintenir une forme ou une coiffure donnée.

Les polymères fixants utilisables dans la composition cosmétique selon l'invention sont notamment choisis parmi les polymères cationiques, anioniques, amphotères, non ioniques et leurs mélanges.

Par « polymère cationique », on entend au sens de la présente invention, tout polymère comprenant des groupements cationiques et/ou des groupements ionisables en groupements cationiques.

Les polymères fixants cationiques utilisables dans la composition cosmétique selon l'invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 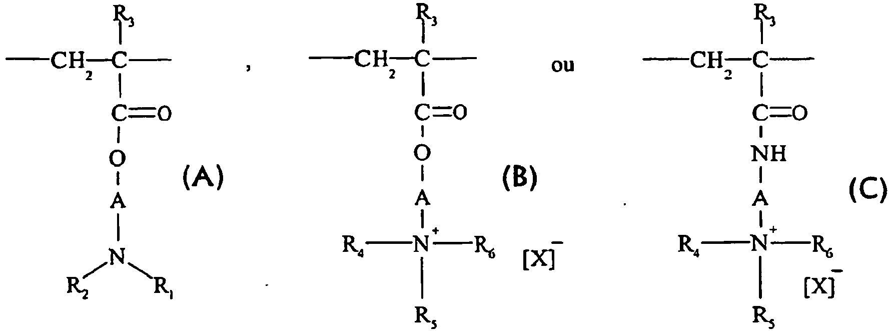 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.

Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁-C₄), des groupes dérivés des acides acryliques ou méthacryliques ou de leurs esters, de vinyllactames tels que la vinylpyrrolidone ou le vinylcaprolactame, d'esters vinyliques.

Ainsi, parmi ces copolymères de la famille (1), on peut citer :
- les copolymères d'acrylamide et de méthacrylate de diméthylaminoéthyle quaternisés au sulfate de diméthyle ou avec un halogénure de diméthyle, tels que celui vendu sous la dénomination HERCOFLOC^{®} par la société HERCULES,
- les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
- les copolymères d'acrylamide et de méthosulfate de méthacryloyloxyéthyltriméthylammonium, tels que celui vendu sous la dénomination RETEN par la société HERCULES,
- les copolymères vinylpyrrolidone/acrylate ou méthacrylate de dialkylaminoalkyle quaternisés ou non, tels que les produits vendus sous la dénomination "GAFQUAT^{®}" par la société ISP comme, par exemple, "GAFQUAT^{®} 734" ou "GAFQUAT^{®} 755", ou bien les produits dénommés "COPOLYMER^{®} 845,958 et 937". Ces polymères sont décrits en détail dans les brevets français Nos 2 077 143 et 2 393 573,
- les terpolymères méthacrylate de diméthylaminoéthyle/vinylcaprolactame/vinylpyrrolidone tels que le produit commercialisé sous la dénomination GAFFIX^{®} VC 713 par la société ISP, et
- les copolymères vinylpyrrolidone/méthacrylamide de diméthylaminopropyle quatemisé tels que notamment le produit commercialisé sous la dénomination "GAFQUAT® HS 100" par la société ISP ;
   (2) les polysaccharides cationiques, de préférence à ammonium quaternaire, tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL ;
   (3) les copolymères quatemaires de vinylpyrrolidone et de vinylimidazole ;
   (4) les chitosanes ou leurs sels ; les sels utilisables sont en particulier l'acétate, le lactate, le glutamate, le gluconate ou le pyrrolidone-carboxylate de chitosane.
      Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER^{®} PC par la société AMERCHOL.
   (5) les dérivés de cellulose cationiques tels que les copolymères de cellulose ou de dérivés de cellulose greffés avec un monomère hydrosoluble comportant un ammonium quaternaire, et décrits notamment dans le brevet US 4131 576, tels que les hydroxyalkylcelluloses, comme les hydroxyméthyl-, hydroxyéthyl-ou hydroxypropyl-celluloses greffées notamment avec un sel de méthacryloyloxyéthyl-triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 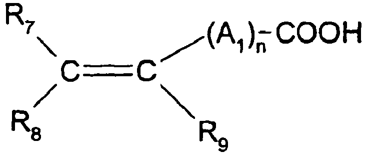 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID, et ULTRAHOLD^{®} par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois nos 75370 et 75371 ou proposés sous la dénomination QUADRAMER par la Société AMERICAN CYANAMID. On peut également citer les terpolymères acide méthacrylique/acrylate d'éthyle/acrylate de tertiobutyle tels que le produit commercialisé sous la dénomination LUVIMER^{®} 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD^{®} DR 25 par la société AMERCHOL.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français nos 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.
D) Les copolymères d'acides ou d'anhydrides carboxyliques monoinsaturés en C₄-C₈, choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ^{®} AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
Ces polymères sont par exemple décrits dans les brevets français nos 2 350 384 et 2 357 241 de la demanderesse. E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
   - les sels de l'acide polystyrène-sulfonique tels que les sels de sodium vendus par exemple sous les dénominations Flexan^{®} 500 et Flexan^{®} 130 par National Starch. Ces composés sont décrits dans le brevet FR 2 198 719.
   - les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA PO-LYMER HSP 1180 par Henkel.

De préférence, les polymères fixants anioniques sont choisis parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/ acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX^{®} A par la société BASF.

Parmi les polymères fixants anioniques cités ci-dessus, on préfère plus particulièrement utiliser dans le cadre de la présente invention les copolymères méthylvinyléther/anhydride maléique monoestérifiées vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désignerdes groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement les copolymères dont la dénomination CTFA (4ème Ed., 1991) est octylacrylamide/ acrylates/ butylaminoethyl methacrylate copolymer, tels que les produits vendus sous la dénomination AMPHOMER^{®} ou LOVOCRYL^{®} 47 par la société NATIONAL STARCH.
(3) les polyaminoamides réticulés et acylés partiellement ou totalement dérivant de polyaminoamides de formule générale : 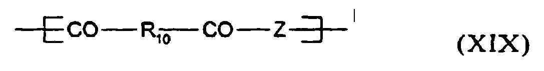 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 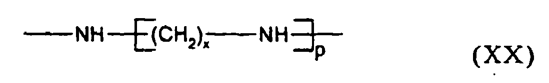 où x=2 et p=2
      ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (XXI) ci-dessus, dans lequel x=2 et p=1 et qui dérive de l'éthylène-diamine, ou le groupe dérivant de la pipérazine : 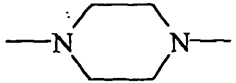
   c) dans les proportions de 0 à 20 % en moles, le groupe -NH-(CH₂)₆-NH- dérivant de l'hexaméthylènediamine, ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.

Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 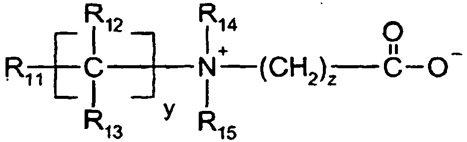 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.

Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.

A titre d'exemple, on peut citer les copolymères méthacrylate de méthyle/diméthyl-carboxyméthylammonioé-thylméthacrylate de méthyle, tels que le produit vendu sous la dénomination DIAFORMER Z301 par la société SANDOZ.
(5) les polymères dérivés du chitosane comportant des motifs monomères répondant aux formules suivantes : 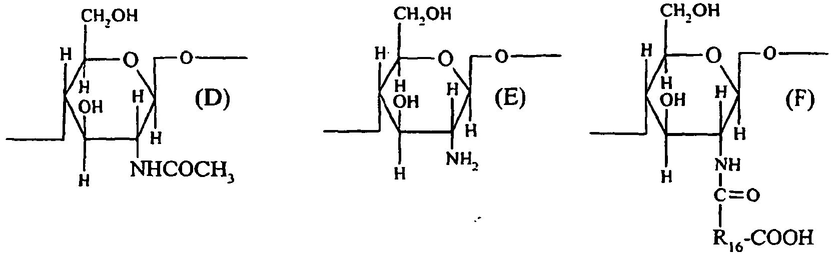 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 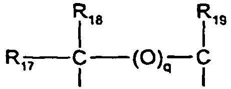 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères comportant des motifs répondant à la formule générale (XXII) sont, par exemple, décrits dans le brevet français 1 400 366 : 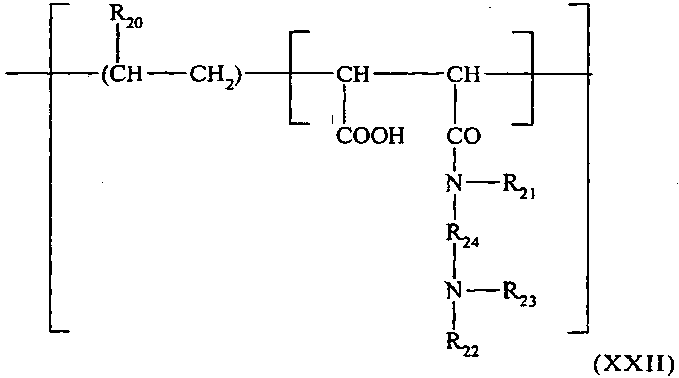 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendu sous la dénomination "EVALSAN" par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (XXIII)

      où D désigne un groupe 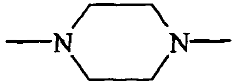 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (XXIV')

      où D désigne un groupe 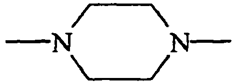
      et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
      (9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N, N-dialkylaminoalcanol. Ces copolymères peuvent également comporter d'autres comonomères vinyliques tels que le vinylcaprolactame.

Parmi les polymères fixants amphotères décrits ci-dessus, les plus particulièrement préférés selon l'invention sont ceux de la famille (3)tels que les copolymères dont la dénomination CTFA est Octylacrylamide/acrylates/butylaminoethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthylcarboxyméthylammonioéthylméthacrylate de méthyle vendu par exemple sous la dénomination DIAFORMER^{®} Z301 par la société SANDOZ.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth) acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHODIA CHIMIE ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyle des polymères non ioniques mentionnés ci-dessus ont, de préférence, de 1 à 6 atomes de carbone.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR^{®} et LUVISET^{®} Si PUR par la société BASF.

Le ou les polymères fixants additionnels est ou sont présents dans la composition cosmétique selon l'invention en une quantité variant de 0,01 à 20 % en poids, de préférence de 0,05 à 15 % en poids, et encore plus préférentiellement de 0,1 à 10 % en poids, par rapport au poids total de la composition cosmétique.

La composition cosmétique selon l'invention peut en outre comprendre un ou plusieurs polymères épaississants encore appelé(s) "agents d'ajustement de la rhéologie".

Les agents d'ajustement de la rhéologie peuvent être choisis parmi les amides d'acides gras (diéthanol- ou monoéthanol-amide de coprah, monoéthanolamide d'acide alkyl éther carboxylique oxyéthyléné), les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs tels que décrits ci-dessous autres que les polymères de l'invention.

Les polymères associatifs utilisables dans la composition cosmétique selon l'invention sont des polymères hydrosolubles capables, dans un milieu aqueux, de s'associer réversiblement entre eux ou avec d'autres molécules.

Leur structure chimique comprend des zones hydrophiles, et des zones hydrophobes caractérisées par au moins une chaîne grasse.

Les polymères associatifs utilisables selon l'invention peuvent être de type anionique, cationique, amphotère et de préférence non ionique.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (XXV) suivante :

   CH₂ = C R'CH₂OBₙR (XXV)

   dans laquelle R' désigne H ou CH₃, 8 désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (XXV) plus particulièrement préféré est un motif dans lequel R' désigne H, n est égal à 10, et R désigne un radical stéaryl (C₁₈).

Des polymères associatifs anioniques de ce type sont décrits et préparés, selon un procédé de polymérisation en émulsion, dans le brevet EP-0 216 479.

Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (XXV), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC80^{®} et SALCARE SC90^{®} qui sont des émulsions aqueuses à 30% d'un terpolymère réticulé d'acide méthacrylique, d'acrylate d'éthyle et de steareth-10-allyl éther (40/50/10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (XXVI) suivante : 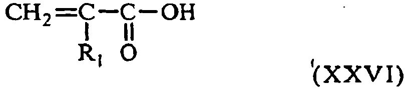 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (XXVII) suivante 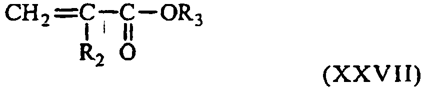 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Parmi ce type de polymères associatifs anioniques, on utilisera plus particulièrement des polymères formés à partir d'un mélange de monomères comprenant :
(i) essentiellement de l'acide acrylique,
(ii) un ester de formule (XXVII) décrite ci-dessus et dans laquelle R₂ désigne H ou CH₃, R₃ désignant un radical alkyle ayant de 12 à 22 atomes de carbone, et
(iii) un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TR1®, PEMULEN TR2®, CAR-BOPOL 1382®, et encore plus préférentiellement le PEMULEN TR1®, et le produit vendu par la société S.E.P.P.I.C. sous la dénomination COATEX SX®.
- (III) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- (IV) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (400E) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄,

A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

Parmi les polymères associatifs de type cationique, on peut citer:
- (I) les polyuréthanes associatifs cationiques dont la famille a été décrite par la demanderesse dans la demande de brevet français N° 0009609; elle peut être représentée par la formule générale (XXVIII) suivante :

   R-X-(P)n-[L-(Y)m]r-L'-(P')p-X'-R' (XXVIII)

   dans laquelle :
   R et R', identiques ou différents, représentent un groupement hydrophobe ou un atome d'hydrogène ;
   X et X', identiques ou différents, représentent un groupement comportant une fonction amine portant ou non un groupement hydrophobe, ou encore le groupement L" ;
   L, L' et L", identiques ou différents, représentent un groupement dérivé d'un diisocyanate ;
   P et P', identiques ou différents, représentent un groupement comportant une fonction amine portant ou non un groupement hydrophobe ;
   Y représente un groupement hydrophile ;
   r est un nombre entier compris entre 1 et 100, de préférence entre 1 et 50 et en particulier entre 1 et 25,
   n, m, et p valent chacun indépendamment des autres entre 0 et 1000 ;
   la molécule contenant au moins une fonction amine protonée ou quaternisée et au moins un groupement hydrophobe.

Dans un mode de réalisation préféré de ces polyuréthanes, les seuls groupements hydrophobes sont les groupes R et R' aux extrémités de chaîne.

Une famille préférée de polyuréthanes associatifs cationiques est celle correspondant à la formule (XIX) décrite ci-dessus et dans laquelle:
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (XIX) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.
Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (XIX) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 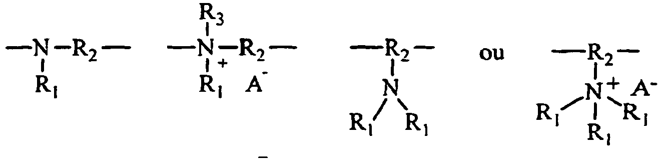 pour X 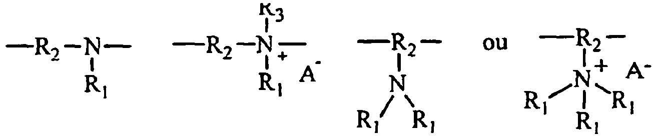 pour X'
dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 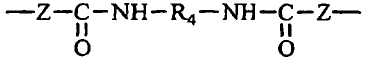 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 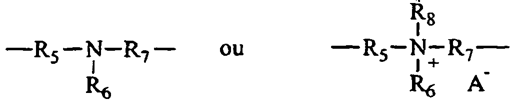 ou 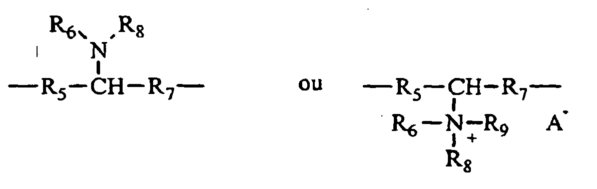 ou 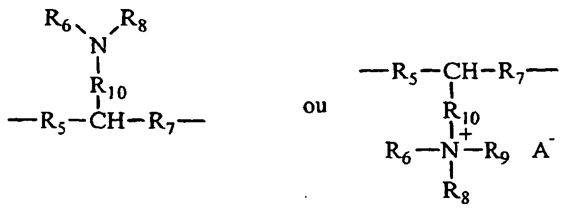 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (XXVIII) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (XXVIII) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)n-ZH,

ou

HZ-(P')p-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (XXVIII) est un diisocyanate correspondant à la formule :

O=C=N-R₄-N=C=O

dans laquelle R₄ est défini plus haut.

A titre d'exemple, on peut citer le méthylènediphényldiisocyanate, le méthylènecyclohexanedïïsocyanate, l'isophoronediisocyanate, le toluènediisocyanate, le naphtalènediisocyanate, le butanediisocyanate, l'hexanediisocyanate.

Un troisième composé entrant dans la préparation du polyuréthane de formule (XXVIII) est un composé hydrophobe destiné à former les groupes hydrophobes terminaux du polymère de formule (XXVIII).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.

Le groupe hydrophobe du polyuréthane de formule (XXVIII) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (XXVIII) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.
- (II) les dérivés de cellulose quaternisée et les polyacrylates à groupements latéraux aminés non cycliques.

Les dérivés de cellulose quaternisée sont en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C12) et CRODACEL QS® (alkyle en C₁₈) commercialisés par la société CRODA.

Les polymères associatifs amphotères sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant :
1) au moins un monomère de formule (XXIX) ou (XXX) :
   A⁻ 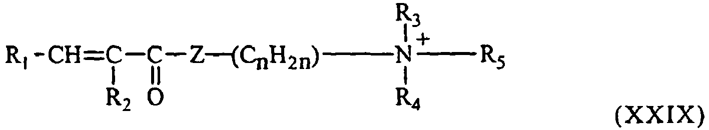 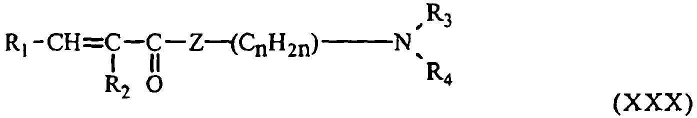 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
      Z représente un groupe NH ou un atome d'oxygène,
      n est un nombre entier de 2 à 5,
      A⁻ est un anion issu d'un acide organique ou minéral, tel qu'un anion méthosulfate ou un halogénure tel que chlorure ou bromure;
2) au moins un monomère de formule (XXXI)

   R₆ -CH =CR₇ -COOH (XXXI)

   dans laquelle, R₆ et R₇, identiques ou différents, représentent un atome d'hydrogène ou un radical méthyle;
   et
3) au moins un monomère de formule (XXXIII) :

   R₆-CH =CR₇ -COXR₈ (XXXIII)

   dans laquelle R₆ et R₇, identiques ou différents, représentent un atome d'hydrogène ou un radical méthyle, X désigne un atome d'oxygène
   ou d'azote et R₈ désigne un radical alkyle linéaire ou ramifié ayant de 1 à 30 atomes de carbone ;
   l'un au moins des monomères de formule (XXIX), (XXX) ou (XXXIII) comportant au moins une chaîne grasse.

Les monomères de formule (XXIX) et (XXX) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide, ces monomères étant éventuellement quaternisés, par exemple par un halogénure d'alkyle en C₁-C₄ ou un sulfate de dialkyle en C₁-C₄.

Plus particulièrement, le monomère de formule (XXIX) est choisi parmi le chlorure d'acrylamidopropyl triméthyl ammonium et le chlorure de méthacrylamidopropyl triméthyl ammonium.

Les monomères de formule (XXXI) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XXXI) est l'acide acrylique.

Les monomères de formule (XXXIII) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quatemisés.

Le rapport du nombre de charges cationiques/charges anioniques est de préférence égal à environ 1.

Les polymères associatifs amphotères selon l'invention comprennent de préférence de 1 à 10 % moles du monomère comportant une chaîne grasse (monomère de formule (XXIX), (XXX) ou (XXXIII)), et de préférence de 1,5 à 6% moles.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autres monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO98/44012.

Parmi les polymères associatifs amphotères selon l'invention, on préfère les terpolymères acide acrylique/ chlorure de (méth)acrylamidopropyl triméthyl ammonium/ méthacrylate de stéaryle.

Les polymères associatifs de type non ionique utilisables selon l'invention sont choisis de préférence parmi :
- (1) les celluloses modifiées par des groupements comportant au moins une chaîne grasse ; on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS G RADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212 ainsi que l'Acrysol RM 184®.

On peut également citer le produit ELFACOS T210® à chaîne alkyle en C₁₂₋₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Le ou les polymères épaississants additionnel(s) est ou sont présents dans la composition cosmétique selon l'invention en une quantité variant de 0,01 à 20 % en poids, de préférence en une quantité variant de 0,05 à 10 % en poids et encore plus préférentiellement en une quantité variant de 0,1 à 5 % en poids, par rapport au poids total de la composition.

La composition cosmétique selon l'invention peut comprendre en outre un ou plusieurs adjuvants cosmétiques choisis parmi les agents tensioactifs cationiques, anioniques, amphotères, non ioniques, les silicones autres que celles de l'invention, les agents conditionneurs de type esters, les agents anti-mousse, les agents hydratants, les agents émollients, les plastifiants, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents anti-corrosion, les corps gras tels que les huiles végétales, animales, minérales et synthétiques, les agents réducteurs ou antioxydants, les agents oxydants.

L'homme du métier veillera à choisir les éventuels adjuvants et leur quantité de manière à ce qu'ils ne nuisent pas aux propriétés des compositions de la présente invention.

De préférence, le ou les adjuvants cosmétiques sont présents à une concentration allant de 0,001 à 50 % en poids par rapport au poids total de la composition.

Par milieu cosmétiquement acceptable, on entend un milieu compatible avec les matières kératiniques et en particulier les cheveux.

Le milieu cosmétiquement acceptable peut être un milieu alcoolique, aqueux ou hydroalcoolique. Ainsi le milieu peut notamment être constitué uniquement par de l'eau ou de l'alcool ou par un mélange d'eau et d'un ou de plusieurs solvants cosmétiquement acceptables tels que les alcools inférieurs en C₁-C₄, les polyols, les monoéthers de polyols et leurs mélanges. De préférence, l'alcool est l'éthanol.

Les compositions cosmétiques conformes à l'invention peuvent se présenter sous forme de crème, de mousse, de lotion, de spray, de gel ou d'après-shampooing.

Les compositions cosmétiques conformes à l'invention peuvent aussi être conditionnés dans un flacon pompe ou encore dans un dispositif aérosol usuel en cosmétique.

Les agents propulseurs utilisés dans les systèmes aérosols selon l'invention peuvent être choisis parmi l'air, l'azote, le gaz carbonique, le diméthyléther, les alcanes en C₃ à C₅, le 1,1-difluoroéthane et leurs mélanges.

La présente invention concerne également un dispositif aérosol comprenant la composition décrite ci-dessus et un moyen de distribution de cette composition.

La présente invention concerne aussi un procédé de traitement cosmétique des cheveux, par exemple de coiffage, qui consiste à appliquer une quantité efficace d'une composition décrite ci-dessus, sur les cheveux secs ou humides, à rincer ou non après un éventuel temps de pose ou après un éventuel séchage.

La présente invention concerne également l'utilisation d'une composition cosmétique pour le soin des cheveux.

L'exemple suivant est donné à titre illustratif.

### EXEMPLE

On a préparé les compositions (A) à (D) conformes à l'invention à partir des composés suivants :

**Composition A**

| | | |
|---|---|---|
| Styleze W20 (1) | | 1 % |
| Alcool cétylique | | 3 % |
| Amodiméthicone (DC 939) | | 1,5 % |
| Chlorure de cétyltriméthylammonium | | 1 % |
| Hydroxypropyl guar | | 0,5 % |
| Stéarate de glycérol | | 1 % |
| Polyvinylpyrrolidone | | 2 % |
| Conservateurs | | qs % |
| Eau | qs | 100 % |
| (1)Terpolymère vinylpyrrolidone /diméthylaminopropylméthacrylamide /chlorure de lauryldiméthylméthacrylamidopropylammonium, proposé par la société ISP sous la dénomination Styleze W20 | | |

**Composition B**

| | | |
|---|---|---|
| Styleze W20 (1) | | 4 % |
| Alcool cétéarylique | | 4 % |
| Glycérol | | 5% |
| Amodiméthicone (DC 939) | | 1,5 % |
| Chlorure de béhényltriméthylammonium | | 1 % |
| Conservateurs | | qs % |
| Eau | qs | 100 % |
| (1)Terpolymère vinylpyrrolidone /diméthylaminopropylméthacrylamide/chlorure de lauryldiméthylméthacrylamidopropylammonium, proposé par la société ISP sous la dénomination Styleze W20 | | |

**Composition C**

| | | |
|---|---|---|
| Styleze W20 (1) | | 5 % |
| Alcool cétéarylique | | 4 % |
| Amodiméthicone (DC 939) | | 1,5 % |
| Chlorure de béhényltriméthylammonium | | 1 % |
| Laureth-4 | | 1 % |
| Huile minérale | | 1 % |
| Conservateurs | | qs % |
| Eau | qs | 100 % |
| (1)Terpolymère vinylpyrrolidone /diméthylaminopropylméthacrylamide/chlorure de lauryldiméthylméthacrylamidopropylammonium, proposé par la société ISP sous la dénomination Styleze W20 | | |

**Composition D**

| | | |
|---|---|---|
| Styleze W20 (1) | | 1 % |
| Alcool cétylique | | 4 % |
| Amodiméthicone (DC 939) | | 1 % |
| Chlorure de cétyltriméthylammonium | | 1 % |
| Diméthiconol (DC 1501 FLUID) | | 2 % |
| Conservateurs | | qs % |
| Eau | qs | 100% |
| (1)Terpolymère vinylpyrrolidone /diméthylaminopropylméthacrylamide /chlorure de lauryldiméthylméthacrylamidopropylammonium, proposé par la société ISP sous la dénomination Styleze W20 | | |

Les pourcentages de chacun des composés dans les compositions cosmétiques selon l'invention sont calculés en poids de matières actives par rapport au poids total de la composition.

Les compositions (A) à (D) sont appliquées sur des cheveux européens.

On constate que les compositions conformes à l'invention permettent de diminuer sensiblement le volume des cheveux frisottés tout en conférant aux cheveux de bonnes propriétés cosmétiques, et en particulier un toucher satisfaisant.

## Revendications

1. Composition cosmétique pour le traitement des fibres kératiniques, en particulier des fibres kératiniques humaines telles que les cheveux, **caractérisée par le fait qu'**elle comprend, dans un milieu cosmétiquement acceptable,
- au moins un polymère poly(vinyllactame) cationique comprenant :
- a) au moins un monomère de type vinyl lactame ou alkylvinyllactame;
- b) au moins un monomère de structures (Ia) ou (Ib) suivantes : 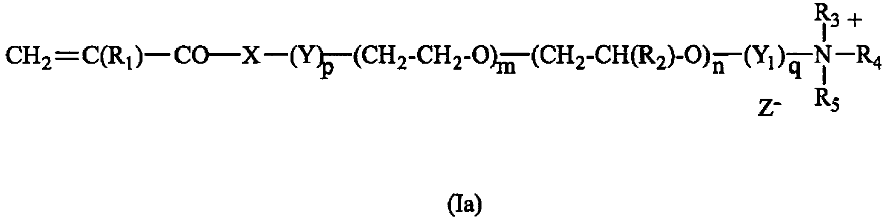 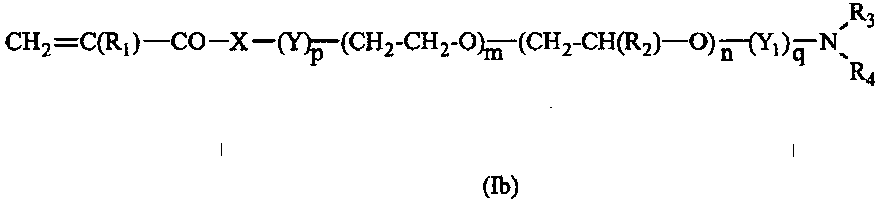 dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄, R₃, R₄ et R₅ désignent, indépendamment l'un de l'autre, un atome d'hydrogène, un radical alkyle linéaire ou ramifié en C₁-C₃₀ ou un radical de formule (II) : 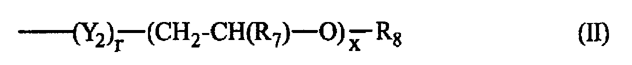
Y, Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0 ;
- au moins un alcool gras présent dans une quantité variant de 0,1 à 30% en poids par rapport au poids total de la composition, et
- au moins une silicone aminée.

2. Composition cosmétique selon la revendication 1, **caractérisée par le fait que** le monomère vinyl lactame ou alkylvinyllactame est un composé de structure (III) : 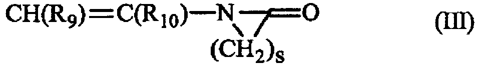 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

3. Composition cosmétique selon la revendication 2, **caractérisée par le fait que** le monomère de formule (III) est la vinylpyrrolidone.

4. Composition cosmétique selon l'une quelconque des revendications précédentes, **caractérisée par le fait que** dans les formules (Ia) ou (Ib), les radicaux R₃, R₄, R₅ désignent, indépendamment l'un de l'autre, un atome d'hydrogène ou un radical alkyle linéaire ou ramifié en C₁-C₃₀.

5. Composition cosmétique selon l'une quelconque des revendications précédentes, **caractérisée par le fait que** le monomère b) est un monomère de formule (Ia).

6. Composition cosmétique selon la revendication 5, **caractérisée par le fait que** dans la formule (Ia), m et n sont égaux à zéro.

7. Composition cosmétique selon l'une quelconque des revendications précédentes, **caractérisée par le fait que** le contre ion Z⁻ des monomères de formule (Ia) est choisi parmi les ions halogénures, les ions phosphates, l'ion méthosulfate, l'ion tosylate.

8. Composition cosmétique selon l'une quelconque des revendications 1 à 7, **caractérisée par le fait que** le ou les polymères poly(vinyllactame) cationiques contiennent un ou plusieurs monomères supplémentaires cationiques ou non ioniques.

9. Composition cosmétique selon la revendication 8, **caractérisée par le fait que** le poly(vinyllactame) cationique est un terpolymère comprenant :
a)-un monomère de formule (III),
b)-un monomère de formule (Ia) dans laquelle p=1, q=0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (Ib) dans laquelle R₃ et R₄ désignent, indépendamment l'un de l'autre, un atome d'hydrogène ou un radical alkyle en C₁-C₅.

10. Composition cosmétique selon la revendication 9, **caractérisée par le fait que** le terpolymère comprend en poids, 40 à 95% de monomère (a), 0,25 à 50% de monomère (b) et 0,1 à 55% de monomère (c).

11. Composition selon l'une quelconque des revendications précédentes, **caractérisée par le fait que** les poly(vinyllactame) cationiques sont choisis parmi les terpolymères vinylpyrrolidone / diméthylaminopropylméthacrylamide / tosylate de dodécyldiméthylméthacrylamidopropylammonium, les terpolymères vinylpyrrolidone / diméthylaminopropylméthacrylamide / tosylate de cocoyldiméthylméthacrylamidopropylammonium, les terpolymères vinylpyrrolidone / diméthylaminopropylméthacrylamide / tosylate ou chlorure de lauryldiméthylméthacrylamidopropylammonium.

12. Composition selon l'une quelconque des revendications précédentes, **caractérisée par le fait que** ledit polymère poly(vinyllactame) cationique est un terpolymère vinylpyrrolidone / diméthylaminopropylméthacrylamide / chlorure de lauryldiméthylméthacrylamidopropylammonium.

13. Composition selon l'une quelconque des revendications précédentes, **caractérisée par le fait que** la masse moléculaire en poids des poly(vinyllactame) cationiques est comprise entre 500 et 20 000 000, de préférence comprise entre 200 000 et 2 000 000 et plus préférentiellement comprise entre 400 000 et 800 000.

14. Composition cosmétique selon l'une quelconque des revendications précédentes, **caractérisée par le fait que** le ou les poly(vinyllactame) cationiques sont utilisés en une quantité variant de 0,05 à 30 % en poids, de préférence en une quantité variant de 0,1 à 15 % en poids et encore plus préférentiellement en une quantité variant de 0,2 à 10 % en poids, par rapport au poids total de la composition.

15. Composition cosmétique selon l'une quelconque des revendications précédentes, **caractérisée par le fait que** l'alcool gras est linéaire ou ramifié, saturé ou insaturé.

16. Composition cosmétique selon la revendication 15, **caractérisée par le fait que** ledit alcool gras non oxyalkyléné présente la structure R-OH, dans laquelle R désigne un radical saturé ou insaturé, linéaire ou ramifié, comportant de 8 à 40 atomes de carbone et de préférence de 8 à 30 atomes de carbone.

17. Composition cosmétique selon la revendication 16, **caractérisée par** le fait ledit alcool gras est choisi parmi les alcools laurique, cétylique, stéarylique, oléïque, béhénique, linoléique, undécylénique, palmitoléïque, arachidonique, érucique, cétéarylique, et leurs mélanges.

18. Composition cosmétique selon la revendication 17, **caractérisée par le fait que** ledit alcool gras est choisi parmi les alcools cétylique et cétéarylique.

19. Composition cosmétique selon l'une quelconque des revendications précédentes, **caractérisée par le fait que** l'alcool gras est solide ou pâteux à 25°C.

20. Composition cosmétique selon l'une quelconque des revendications précédentes, **caractérisée par le fait que** le ou les alcools gras sont utilisés en une quantité variant de 0,2 à 20 % en poids, et encore plus préférentiellement de 0,5 à 10 % en poids, par rapport au poids total de la composition.

21. Composition cosmétique selon l'une quelconque des revendications précédentes, **caractérisée par le fait que** la silicone aminée est choisie parmi :
a) les composés répondant à la formule (V) suivante :
(R¹)ₐ(T)₃₋ₐ-Si[OSi(T)₂)ₙ-[OSi(T)_{b}(R¹)_{2-b}]ₘ-OSi(T)₃₋ₐ-(R¹)ₐ
dans laquelle : T est un atome d'hydrogène, ou un radical phényle, hydroxyle, ou alkyle en C₁-C₈, et de préférence méthyle, ou alcoxy en C₁-C₈, de préférence méthyle,
a désigne le nombre 0 ou un nombre entier de 1 à 3, et de préférence 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R¹ est un radical monovalent de formule -C_{q}H_{2q}L dans laquelle q est un nombre de 2 à 8 et L est un groupement aminé éventuellement quaternisé choisi parmi les groupements :
- N(R²)-CH₂-CH₂-N(R²)₂ ;
- N(R²)₂ ;
- N⁺(R²)₃ Q⁻ ;
- N⁺(R²) (H)₂ Q⁻ ;
- N⁺(R²)₂HQ⁻ ;
- N(R²)-CH₂-CH₂-N⁺(R²)(H)₂ Q⁻,
dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.
b) les silicones répondant à la formule (VII) suivante : 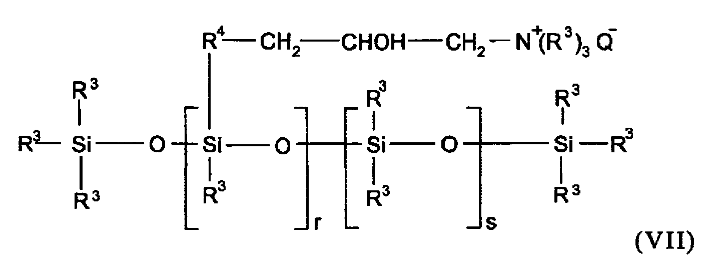 dans laquelle,
R³ représente un radical hydrocarboné monovalent en C₁-C₁₈, et en particulier un radical alkyle en C₁-C₁₈, ou alcényle en C₂-C₁₈, par exemple méthyle ;
R⁴ représente un radical hydrocarboné divalent, notamment un radical alkylène en C₁-C₁₈ ou un radical alkylèneoxy divalent en C₁-C₁₈, par exemple en C₁-C₈;
Q- est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.
c) les silicones ammonium quaternaire de formule (XII): 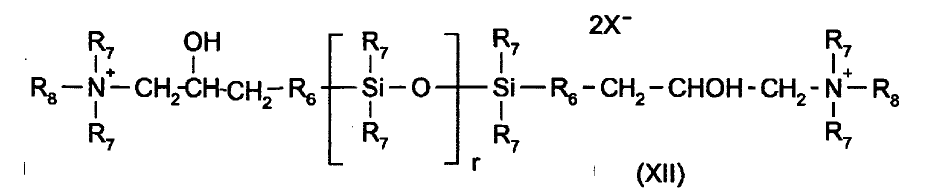 dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone ;
R₆ représente un radical hydrocarboné divalent, notamment un radical alkylène en C₁-C₁₈ ou un radical alkylèneoxy divalent en C₁-C₁₈;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
X- est un anion tel qu'un ion halogénure ou un sel d'acide organique;
r représente une valeur statistique moyenne de 2 à 200 et en particulier de 5 à 100 ;
d) les silicones aminées de formule (XIII) : 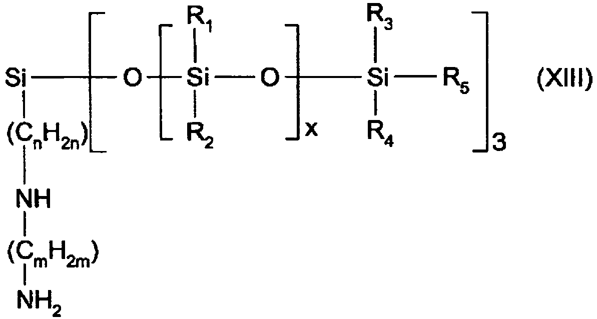 dans laquelle :
- R₁, R₂, R₃ et R₄, identiques ou différents, désignent un radical alkyle en C₁-C₄ ou un groupement phényle,
- R₅ désigne un radical alkyle en C₁-C₄ ou un groupement hydroxyle,
- n est un entier variant de 1 à 5,
- m est un entier variant de 1 à 5,
et dans laquelle x est choisi de manière telle que l'indice d'amine soit compris entre 0,01 et 1 meq/g.

22. Composition cosmétique selon la revendication 21, **caractérisée par le fait que** la silicone aminée correspondant à la formule (V) est choisie parmi les composés correspondant à la formule suivante : 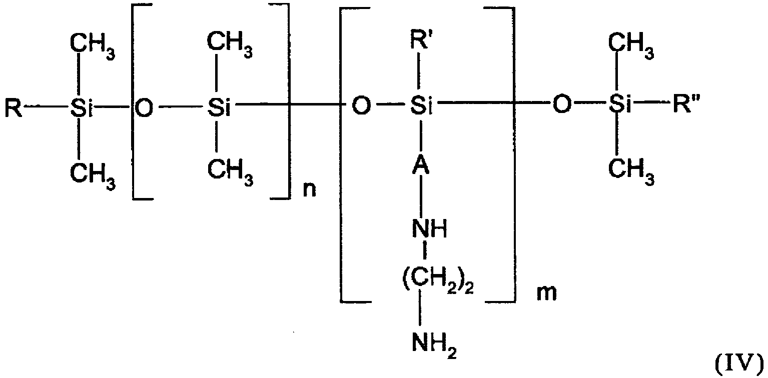 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

23. Composition cosmétique selon la revendication 21, **caractérisée par le fait que** la silicone aminée de formule (V) est la silicone dénommée « triméthylsilylamodiméthicone », répondant à la formule (VI) suivante : 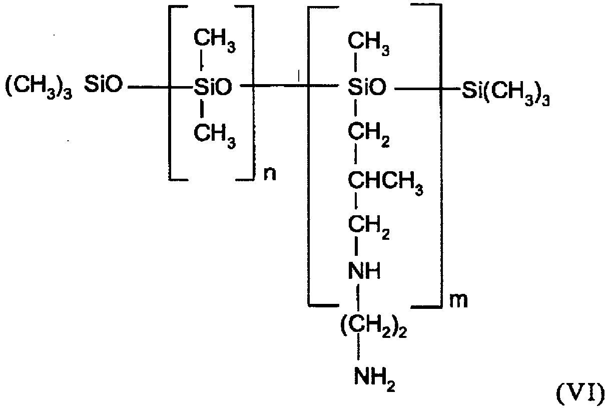 dans laquelle n et m ont les significations indiquées à la formule (V).

24. Composition cosmétique selon l'une quelconque des revendications précédentes, **caractérisée par le fait que** le ou les silicones aminées est ou sont présentes dans une quantité variant de 0,01 à 20 % en poids, de préférence dans une quantité variant de 0,05 à 10 % en poids, et encore plus préférentiellement dans une quantité variant de 0,1 à 5 % en poids, par rapport au poids total de la composition.

25. Composition cosmétique selon l'une quelconque des revendications précédentes, **caractérisée par le fait que** le rapport pondéral polyvinyllactame cationique / alcool gras est compris entre 0.5 et 5, le rapport pondéral polyvinyllactame cationique / silicone aminée est compris entre 0,5 et 5 et le rapport pondéral silicone aminée / alcool gras est compris entre 0,5 et 10.

26. Composition cosmétique selon l'une quelconque des revendications précédentes, **caractérisée par le fait qu'**elle comprend en outre au moins une silicone oxyalkylénée.

27. Composition cosmétique selon la revendication 26, **caractérisée par le fait que** la silicone oxyalkylénée est choisie parmi les composés de formules générales (XIV), (XV), (XVI), (XVII) ou (XVIII) suivantes: 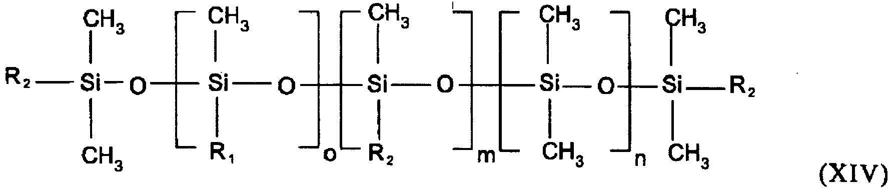 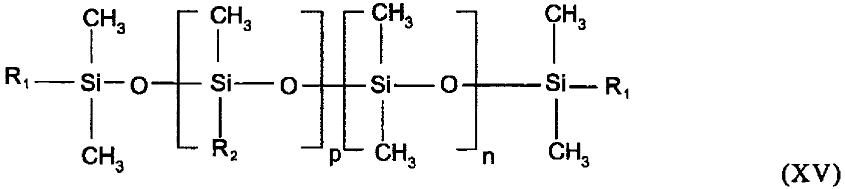 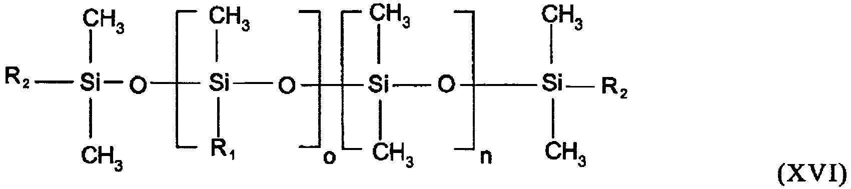 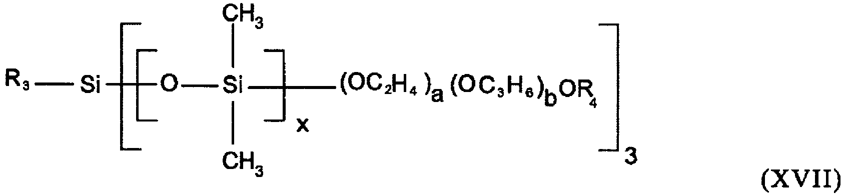 dans lesquelles:
- R₁, identique ou différent, représente un radical alkyle, linéaire ou ramifié, en C₁-C₃₀ ou phényle,
- R₂, identique ou différent, représente un radical -C_{c}H_{2c}-O-(C₂H₄O)ₐ(C₃H₆O)_{b}-R₅ ou un radical -C_{c}H_{2c}-O-(C₄H₈0)ₐ-R₅,
- R₃, R₄, identiques ou différents, désignent un radical alkyle, linéaire ou ramifié, en C₁ à C₁₂, et de préférence le radical méthyle,
- R₅, identique ou différent, est choisi parmi un atome d'hydrogène, un radical alkyle, linéaire ou ramifié, comprenant de 1 à 12 atomes de carbone, un radical alcoxy, linéaire ou ramifié, comprenant de 1 à 6 atomes de carbone, un radical acyle, linéaire ou ramifié, comprenant de 2 à 30 atomes de carbone, un radical hydroxyle, -SO₃M, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement éventuellement substitué par un ou deux radicaux aminoalkyle substitués, -CO(CH₂)_{d}COOM, -COCHR₇(CH₂)_{d}COOM, -NHCO(CH₂)_{d}OH, -NH₃Y, un groupement phosphate,
- M, identique ou différent, désigne un atome d'hydrogène, Na, K, Li, NH₄ ou une amine organique,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 100,
- Y représente un anion minéral ou organique monovalent tel que halogénure (chlorure, bromure), sulfate, carboxylate (acétate, lactate, citrate),
sous réserve que lorsque la silicone est de formule (XV) avec R₅ désignant hydrogène alors n est supérieur à 12,
([ Z (R₂SiO)_{q} R'₂SiZO][(CₙH₂ₙO)ᵣ])ₛ (XVIII)
formule (XVIII,) dans laquelle:
- R₂ et R'₂, identiques ou différents, représentent un radical hydrocarboné monovalent en C₁-C₃₀,
- n est un nombre entier allant de 2 à 4,
- q est un nombre supérieur ou égal à 4, de préférence compris entre 4 et 200 et encore plus particulièrement entre 4 et 100.
- r est un nombre supérieur ou égal à 4, de préférence compris entre 4 et 200 et encore plus particulièrement entre 5 et 100.
- s est un nombre supérieur ou égal à 4, de préférence compris entre 4 et 1 000 et encore plus particulièrement entre 5 et 300.
- Z représente un groupe organique divalent qui est lié à l'atome de silicium adjacent par une liaison carbone-silicium et au bloc polyoxyalkylène (CₙH₂ₙO) par un atome d'oxygène,
- le poids moléculaire moyen de chaque bloc siloxane est compris entre environ 400 et environ 10 000, celui de chaque bloc polyoxyalkylène étant compris entre environ 300 et environ 10 000,
- les blocs siloxane représentent de 10% environ à 95% environ en poids du copolymère bloc,
- le poids moléculaire moyen en nombre du copolymère bloc pouvant aller de 2 500 à 1 000 000.

28. Composition cosmétique selon l'une quelconque des revendications précédentes, **caractérisée par le fait qu'**elle comprend en outre au moins une silicone choisie parmi les gommes de silicone.

29. Composition cosmétique selon la revendication 28, **caractérisée par le fait que** les gommes de silicone sont choisies parmi les polyorganosiloxanes ayant des masses moléculaires moyennes en nombre comprises entre 200 000 et 1000000 utilisés seuls ou sous forme de mélange dans un solvant.

30. Composition cosmétique selon l'une quelconque des revendications 28 ou 29, **caractérisée par le fait que** les gommes de silicone utilisées seules ou sous forme de mélange sont choisies parmi les structures suivantes :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane et les mélanges suivants :
- des mélanges formés à partir d'un polydiméthylsiloxane hydroxylé en bout de chaîne et d'un polydiméthylsiloxane cyclique ;
- des mélanges formés à partir d'une gomme polydiméthylsiloxane et d'une silicone cyclique ;
- des mélanges de polydiméthylsiloxanes de viscosités différentes.

31. Composition cosmétique selon l'une quelconque des revendications précédentes, **caractérisée par le fait qu'**elle comprend en outre au moins un polymère fixant additionnel.

32. Composition cosmétique selon l'une quelconque des revendications précédentes, **caractérisée par le fait qu'**elle comprend en outre au moins un polymère épaississant additionnel.

33. Composition cosmétique selon l'une quelconque des revendications précédentes, **caractérisée par le fait qu'**elle comprend en outre au moins un adjuvant cosmétique choisi parmi les agents tensioactifs cationiques, anioniques, amphotères, non ioniques, les silicones autre que ceux de l'invention, les agents conditionneurs de type esters, les agents anti-mousse, les agents hydratants, les agents émollients, les plastifiants, les filtres solaires hydrosolubles et liposolubles, siliconés ou non siliconés, les colorants permanents ou temporaires, les parfums, les peptisants, les conservateurs, les céramides, et pseudo-céramides, les vitamines et les provitamines dont le panthénol, les protéines, les agents séquestrants, les agents solubilisants, les agents alcanisants, les agents anti-corrosion, les corps gras tels que les huiles végétales, animales, minérales et synthétiques, les agents réducteurs ou antioxydants, les agents oxydants.

34. Composition cosmétique selon la revendication 33, **caractérisée par le fait que** le ou les adjuvants cosmétiques sont présents à une concentration allant de 0,001 à 50 % en poids par rapport au poids total de la composition.

35. Composition cosmétique selon l'une quelconque des revendications précédentes, **caractérisée par le fait que** le milieu cosmétiquement acceptable est un milieu aqueux, alcoolique ou hydroalcoolique.

36. Composition cosmétique selon la revendication 35, **caractérisée par le fait que** le milieu hydroalcoolique comprend les alcools inférieurs en C₁-C₄, les polyols, les monoéthers de polyols et leurs mélanges.

37. Composition cosmétique selon la revendication 36, **caractérisée par le fait que** l'alcool est de l'éthanol.

38. Composition cosmétique selon l'une quelconque des revendications précédentes, **caractérisée par le fait qu'**elle se présente sous forme de crème, de mousse, lotion, de gel, de spray ou d'après-shampooing.

39. Composition cosmétique selon l'une quelconque des revendications précédentes, **caractérisée par le fait qu'**elle est conditionnée dans un vaporisateur, un flacon pompe ou un dispositif aérosol.

40. Composition cosmétique selon la revendication 39, **caractérisée par le fait qu'**elle est conditionnée dans un dispositif aérosol.

41. Composition cosmétique selon la revendication 40, **caractérisée par le fait qu'**elle comprend un agent propulseur choisi parmi l'air, l'azote, le gaz carbonique, le diméthyléther, les alcanes en C₃ à C₅, le 1,1-difluoroéthane et leurs mélanges.

42. Dispositif aérosol formé par un récipient comprenant une composition selon l'une quelconque des revendications précédentes ainsi qu'un moyen de distribution de la composition.

43. Procédé de traitement cosmétique **caractérisée par le fait qu'**il comprend l'application d'une composition cosmétique selon l'une quelconque des revendications 1 à 41, en particulier sur les cheveux.

44. Procédé de traitement cosmétique selon la revendication 43, **caractérisée par le fait que** l'application de ladite composition n'est pas suivie par un rinçage.

45. Utilisation d'une composition cosmétique selon l'une quelconque des revendications 1 à 41 pour le soin des cheveux.

## Patentansprüche

1. Kosmetische Zusammensetzung für die Behandlung von Keratinfasern, insbesondere von menschlichen Keratinfasern wie dem Haar, **dadurch gekennzeichnet, dass** sie in einem kosmetisch unbedenklichen Medium Folgendes umfasst:
- mindestens ein kationisches Poly(vinyllactam)polymer, das Folgendes umfasst:
- a) mindestens ein Monomer des Vinyllactam- oder Alkylvinyllactamtyps;
- b) mindestens ein Monomer mit den folgenden Strukturen (Ia) oder (Ib): 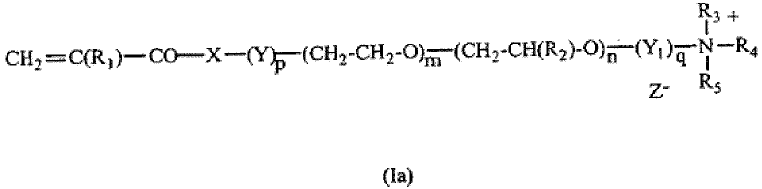 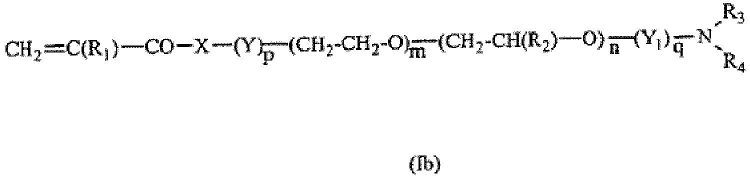 in denen:
X ein Sauerstoffatom oder einen NR₆-Rest bedeutet,
R₁ und R₆ unabhängig voneinander ein Wasserstoffatom oder einen geradkettigen oder verzweigten C₁-C₅-Alkylrest bedeuten,
R₂ einen geradkettigen oder verzweigten C₁-C₄-Alkylrest bedeutet,
R₃, R₄ und R₅ unabhängig voneinander ein Wasserstoffatom, einen geradkettigen oder verzweigten C₁-C₃₀-Alkylrest oder einen Rest der Formel (II) 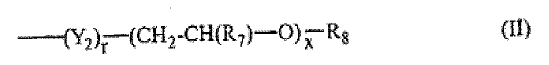 bedeuten,
Y, Y₁ und Y₂ unabhängig voneinander einen geradkettigen oder verzweigten C₂-C₁₆-Alkylenrest bedeuten,
R₇ ein Wasserstoffatom, einen geradkettigen oder verzweigten C₁-C₄-Alkylrest oder einen geradkettigen oder verzweigten C₁-C₄-Hydroxyalkylrest bedeutet,
R₈ ein Wasserstoffatom oder einen geradkettigen oder verzweigten C₁-C₃₀-Alkylrest bedeutet,
p, q und r unabhängig voneinander den Wert 0 oder den Wert 1 bedeuten,
m und n unabhängig voneinander eine ganze Zahl von 0 bis 100 bedeuten,
x eine ganze Zahl von 1 bis 100 bedeutet,
Z ein Anion einer organischen oder anorganischen Säure bedeutet,
mit der Maßgabe, dass:
- mindestens einer der Substituenten R₃, R₄, R₅ oder R₈ einen geradkettigen oder verzweigten C₉-C₃₀-Alkylrest bedeutet,
- wenn m oder n nicht null bedeuten, dann q 1 bedeutet,
- wenn m oder n null bedeuten, dann p oder q 0 bedeutet;
- mindestens einen Fettalkohol, der oder die in einer Menge von 0,1 bis 30 Gew.-% in Bezug auf das Gesamtgewicht der Zusammensetzung eingesetzt wird/werden, und
- mindestens ein Aminosilikon.

2. Kosmetische Zusammensetzung nach Anspruch 1, **dadurch gekennzeichnet, dass** es sich bei dem Vinyllactam- oder Alkylvinyllactammonomer um eine Verbindung der folgenden Struktur (III) handelt: 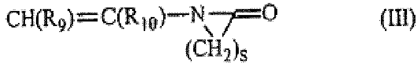 in der
s eine ganze Zahl von 3 bis 6 bedeutet,
R₉ ein Wasserstoffatom oder einen C₁-C₅-Alkylrest bedeutet,
R₁₀ ein Wasserstoffatom oder einen C₁-C₅-Alkylrest bedeutet,
mit der Maßgabe, dass mindestens einer der Reste R₉ und R₁₀ ein Wasserstoffatom bedeutet.

3. Kosmetische Zusammensetzung nach Anspruch 2, **dadurch gekennzeichnet, dass** es sich bei dem Monomer der Formel (III) um Vinylpyrrolidon handelt.

4. Kosmetische Zusammensetzung nach einem der vorhergehenden Ansprüche, **dadurch gekennzeichnet, dass** die Reste R₃, R₄, R₅ in den Formeln (Ia) bzw. (Ib) unabhängig voneinander ein Wasserstoffatom oder einen geradkettigen oder verzweigten C₁-C₃₀-Alkylrest bedeuten.

5. Kosmetische Zusammensetzung nach einem der vorhergehenden Ansprüche, **dadurch gekennzeichnet, dass** es sich bei dem Monomer b) um ein Monomer der Formel (Ia) handelt.

6. Kosmetische Zusammensetzung nach Anspruch 5, **dadurch gekennzeichnet, dass** m und n in Formel (Ia) null bedeuten.

7. Kosmetische Zusammensetzung nach einem der vorhergehenden Ansprüche, **dadurch gekennzeichnet, dass** das Gegenion Z⁻ der Monomere der Formel (Ia) aus der Reihe Halogenidionen, Phosphationen, Methosulfation, Tosylation ausgewählt ist.

8. Kosmetische Zusammensetzung nach einem der Ansprüche 1 bis 7, **dadurch gekennzeichnet, dass** das kationische Poly(vinyllactam)polymer bzw. die kationischen Poly(vinyllactam)polymere ein oder mehrere zusätzliche kationische oder nichtionische Monomere enthalten.

9. Kosmetische Zusammensetzung nach Anspruch 8, **dadurch gekennzeichnet, dass** es sich bei dem kationischen Poly(vinyllactam) um ein Terpolymer handelt, das Folgendes umfasst:
a)- ein Monomer der Formel (III),
b)- ein Monomer der Formel (Ia), worin p=1, q=0, R₃ und R₄ unabhängig voneinander ein Wasserstoffatom oder einen C₁-C₅-Alkylrest bedeuten und R₅ einen C₉-C₂₄-Alkylrest bedeutet sowie
c)- ein Monomer der Formel (Ib), worin R₃ und R₄ unabhängig voneinander ein Wasserstoffatom oder einen C₁-C₅-Alkylrest bedeuten.

10. Kosmetische Zusammensetzung nach Anspruch 9, **dadurch gekennzeichnet, dass** das Terpolymer 40 bis 95 Gew.-% Monomer (a), 0,25 bis 50 Gew.-% Monomer (b) und 0,1 bis 55 Gew.-% Monomer (c) umfasst.

11. Zusammensetzung nach einem der vorhergehenden Ansprüche, **dadurch gekennzeichnet, dass** die kationischen Poly(vinyllactame) aus der Reihe Vinylpyrrolidon/Dimethylaminopropylmethacrylamid/Dodecyldimethylmethacrylamidopropylammoniumtosylat-Terpolymere, Vinylpyrrolidon/Dimethylaminopropylmethacrylamid/Cocoyldimethylmethacrylamidopropylammoniumtosylat-Terpolymere, Vinylpyrrolidon/Dimethylaminopropylmethacrylamid/Lauryldimethylmethacrylamidopropylammoniumtosylat- oder -chlorid-Terpolymere ausgewählt sind.

12. Zusammensetzung nach einem der vorhergehenden Ansprüche, **dadurch gekennzeichnet, dass** es sich bei dem kationischen Poly(vinyllactam)polymer um ein Vinylpyrrolidon/Dimethylaminopropylmethacrylamid/Lauryldimethylmethacrylamidopropylammoniumchlorid-Terpolymer handelt.

13. Zusammensetzung nach einem der vorhergehenden Ansprüche, **dadurch gekennzeichnet, dass** das Gewichts Molekulargewicht der kationischen Poly(vinyllactame) zwischen 500 und 20 000 000, vorzugweise zwischen 200 000 und 2 000 000 und am stärksten bevorzugt zwischen 400 000 und 800 000 beträgt.

14. Kosmetische Zusammensetzung nach einem der vorhergehenden Ansprüche, **dadurch gekennzeichnet, dass** das kationische Poly(vinyllactam) bzw. die kationischen Poly(vinyllactame) in einer Menge von 0,05 bis 30 Gew.-%, vorzugweise in einer Menge von 0,1 bis 15 Gew.-% und noch stärker bevorzugt in einer Menge von 0,2 bis 10 Gew.-% in Bezug auf das Gesamtgewicht der Zusammensetzung eingesetzt wird/werden.

15. Kosmetische Zusammensetzung nach einem der vorhergehenden Ansprüche, **dadurch gekennzeichnet, dass** der Fettalkohol gerade oder verzweigt und gesättigt oder ungesättigt ist.

16. Kosmetische Zusammensetzung nach Anspruch 15, **dadurch gekennzeichnet, dass** der nichtoxyalkenylierte Fettalkohol die Struktur der R-OH aufweist, worin R einen gesättigten oder ungesättigten, geradkettigen oder verzweigten Rest mit 8 bis 40 Kohlenstoffatomen und vorzugweise 8 bis 30 Kohlenstoffatomen bedeutet.

17. Kosmetische Zusammensetzung nach Anspruch 16, **dadurch gekennzeichnet, dass** der Fettalkohol aus der Reihe Laurylalkohol, Cetylalkohol, Stearylalkohol, Oleylalkohol, Behenylalkohol, Linoleylalkohol, Undecylenylalkohol, Palmitoleylalkohol, Arachidonylalkohol, Erucylalkohol und Cetearylalkohol und Mischungen davon ausgewählt ist.

18. Kosmetische Zusammensetzung nach Anspruch 17, **dadurch gekennzeichnet, dass** der Fettalkohol aus der Reihe Cetylalkohol und Cetearylalkohol ausgewählt ist.

19. Kosmetische Zusammensetzung nach einem der vorhergehenden Ansprüche, **dadurch gekennzeichnet, dass** der Fettalkohol bei 25^{°} C fest oder pastös ist.

20. Kosmetische Zusammensetzung nach einem der vorhergehenden Ansprüche, **dadurch gekennzeichnet, dass** der Fettalkohol bzw. die Fettalkohole in einer Menge von 0,2 bis 20 Gew.-% und noch stärker bevorzugt von 0,5 bis 10 Gew.-% in Bezug auf das Gesamtgewicht der Zusammensetzung eingesetzt wird/werden.

21. Kosmetische Zusammensetzung nach einem der vorhergehenden Ansprüche, **dadurch gekennzeichnet, dass** Aminosilikon aus der folgenden Reihe ausgewählt ist:
a) die Verbindungen der folgenden Formel (V):
(R¹)ₐ(T)₃₋ₐ-Si[OSi(T)₂]ₙ-[OSi(T)_{b}(R¹)_{2-b}]ₘ-OSi(T)₃₋ₐ(R¹)ₐ
worin
T ein Wasserstoffatom oder einen Phenyl-, Hydroxyl- oder C₁-C₈-Alkylrest, vorzugsweise einen Methylrest, oder einen C₁-C₈-Alkoxyrest, vorzugsweise Methylrest, bedeutet,
a 0 oder eine ganze Zahl von 1 bis 3, vorzugweise 0, bedeutet,
b 0 oder 1, insbesondere 1, bedeutet,
m und n solche Zahlen bedeuten, dass die Summe (n + m) speziell im Bereich von 1 bis 2 000 und insbesondere im Bereich von 50 bis 150 betragen kann, wobei n eine Zahl von 0 bis 1 999 und insbesondere von 49 bis 149 bedeuten kann und m eine Zahl von 1 bis 2 000, insbesondere von 1 bis 10, bedeuten kann;
R¹ einen einwertigen Rest der Formel -C_{q}H_{2q}L bedeutet, worin q eine Zahl von 2 bis 8 bedeutet und L eine gegebenenfalls quarternisierte Aminogruppe, ausgewählt aus den Gruppen:
- N(R²)-CH₂-CH₂-N(R²)₂ ;
- N(R²)₂ ;
- N⁺(R²)₃ Q⁻ ;
- N⁺(R²) (H)₂ Q⁻ ;
- N⁺(R²)₂HQ⁻ ;
- N(R²)-CH₂-CH₂-N⁺(R²)(H)₂ Q⁻,
bedeutet,
worin R² ein Wasserstoffatom, einen Phenylrest, einen Benzylrest oder einen einwertigen gesättigten Kohlenwasserstoffrest, zum Beispiel einen C₁-C₂₀-Alkylrest, bedeuten kann und Q⁻ ein Halogenidion wie zum Beispiel Fluorid, Chlorid, Bromid oder Iodid bedeutet;
b) Silikone der folgenden Formel (VII): 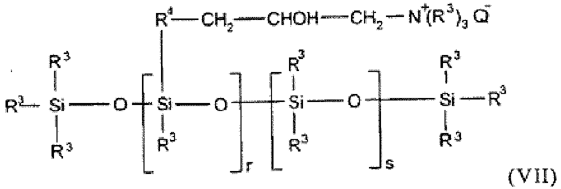 worin
R³ einen einwertigen C₁-C₁₈-Kohlenwasserstoffrest, insbesondere einen C₁-C₁₈-Alkylrest, oder C₂-C₁₈-Alkenylrest, zum Beispiel Methyl, bedeutet;
R⁴ einen zweiwertigen Kohlenwasserstoffrest, insbesondere einen C₁-C₁₈-Alkylenrest oder einen zweiwertigen C₁-C₁₈-Alkylenoxyrest, zum Beispiel C₁-C₈, bedeutet;
Q⁻ ein Halogenidion, insbesondere Chlorid, bedeutet;
r einen durchschnittlichen statistischen Wert von 2 bis 20, insbesondere von 2 bis 8, bedeutet;
s einen mittleren statistischen Wert von 20 bis 200, insbesondere von 20 bis 50, bedeutet;
c) quartäre Ammoniumsilikone der Formel (XII): 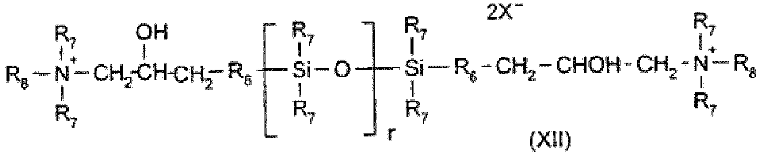 worin
R₇, die gleich oder verschieden sein können, einen einwertigen Kohlenwasserstoffrest mit 1 bis 18 Kohlenstoffatomen, insbesondere einen C₁-C₁₈-Alkylrest, einen C₂-C₁₈-Alkenylrest oder einen Ring mit 5 oder 6 Kohlenstoffatomen bedeuten;
R₆ einen zweiwertigen Kohlenwasserstoffrest, insbesondere einen C₁-C₁₈-Alkylenrest oder einen zweiwertigen C₁-C₁₈-Alkylenoxyrest bedeutet;
R₈, die gleich oder verschieden sind, ein Wasserstoffatom, einen einwertigen Kohlenwasserstoffrest mit 1 bis 18 Kohlenstoffatomen, insbesondere einen C₁-C₁₈-Alkylrest, einen C₂-C₁₈-Alkenylrest, einen Rest -R₆-NHCOR₇ bedeuten;
X- ein Anion wie ein Halogenidion oder ein Salz einer organischen Säure bedeutet;
r einen mittleren statistischen Wert von 2 bis 200, insbesondere von 5 bis 100, bedeutet;
d) Aminosilikone der Formel (XIII): 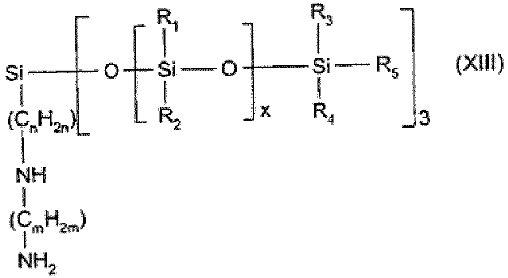 worin
- R₁, R₂, R₃ und R₄, die gleich oder verschieden sind, einen C₁-C₄-Alkylrest oder eine Phenylgruppe bedeuten,
- R₅ einen C₁-C₄-Alkylrest oder eine Hydroxylgruppe bedeutet,
- n eine ganze Zahl von 1 bis 5 bedeutet,
- m eine ganze Zahl von 1 bis 5 bedeutet,
und worin x so gewählt ist, dass die Aminzahl zwischen 0,01 und 1 meq/g liegt.

22. Kosmetische Zusammensetzung nach Anspruch 21, **dadurch gekennzeichnet, dass** das Aminosilikon der Formel (V) aus den Verbindungen der folgenden Formel ausgewählt ist: 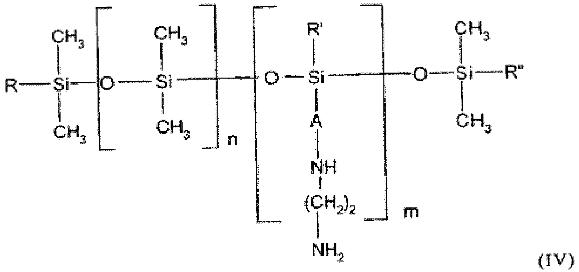 worin
R, R', R'', die gleich oder verschieden sind, einen C₁-C₄-Alkylrest, vorzugsweise CH₃; einen C₁-C₄-Alkoxyrest, vorzugweise Methoxy, oder OH bedeuten; A einen geradkettigen oder verzweigten C₃-C₈-Alkylenrest, vorzugsweise C₃-C₆-Alkylenrest, bedeutet; m und n vom Molekulargewicht abhängige ganze Zahlen, deren Summe zwischen 1 und 2 000 liegt, bedeuten.

23. Kosmetische Zusammensetzung nach Anspruch 21, **dadurch gekennzeichnet, dass** es sich bei dem Aminosilikon der Formel (V) um das Silikon mit der Bezeichnung "Trimethylsilylamodimeticon" der folgenden Formel (VI) handelt: 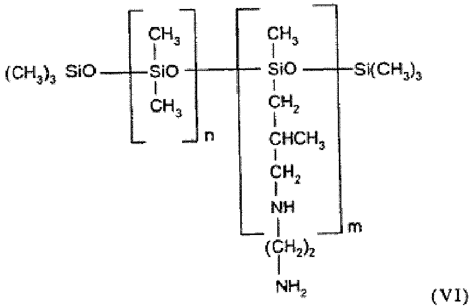 worin n und m die für Formel (V) angegebenen Bedeutungen aufweisen.

24. Kosmetische Zusammensetzung nach einem der vorhergehenden Ansprüche, **dadurch gekennzeichnet, dass** das Aminosilikon bzw. die Aminosilikone in einer Menge von 0,01 bis 20 Gew.-%, vorzugweise in einer Menge von 0,05 bis 10 Gew.-% und noch stärker bevorzugt in einer Menge von 0,1 bis 5 Gew.-% in Bezug auf das Gesamtgewicht der Zusammensetzung vorliegt/vorliegen.

25. Kosmetische Zusammensetzung nach einem der vorhergehenden Ansprüche, **dadurch gekennzeichnet, dass** das Gewichtsverhältnis von kationischem Polyvinyllactam zu Fettalkohol zwischen 0,5 und 5, das Gewichtsverhältnis von kationischem Polyvinyllactam zu Aminosilikon zwischen 0,5 und 5 und das Gewichtsverhältnis von Aminosilikon zu Fettalkohol zwischen 0,5 und 10 liegt.

26. Kosmetische Zusammensetzung nach einem der vorhergehenden Ansprüche, **dadurch gekennzeichnet, dass** sie weiterhin mindestens ein oxyalkenyliertes Silikon umfasst.

27. Kosmetische Zusammensetzung nach Anspruch 26, **dadurch gekennzeichnet, dass** das oxyalkenylierte Silikon aus der Reihe der Verbindungen mit den folgenden allgemeinen Formeln (XIV), (XV), (XVI), (XVII) oder (XVIII) ausgewählt ist: 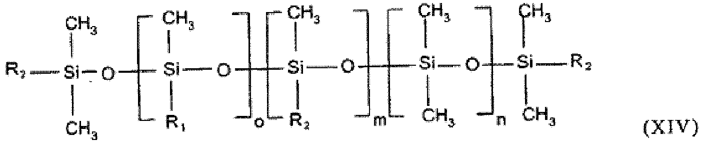 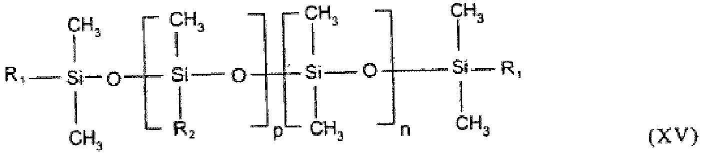 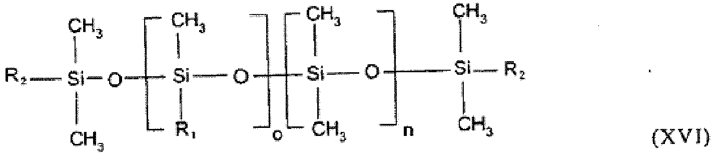 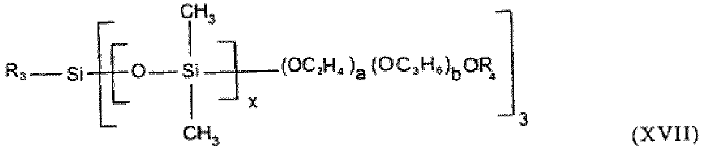 worin
- R₁, die gleich oder verschieden sind, einen geradkettigen oder verzweigten C₁-C₃₀-Alkylrest oder einen Phenylrest bedeuten,
- R₂, die gleich oder verschieden sind, einen Rest -C_{c}H_{2c}-O- (C₂H₄O)ₐ (C₃H₆O)_{b}-R₅ oder einen Rest -C_{c}H_{2c}-O-(C₄H₈O)ₐ-R₅ bedeuten,
- R₃, R₄, die gleich oder verschieden sind, einen geradkettigen oder verzweigten C₁-C₁₂-Alkylrest, vorzugweise den Methylrest, bedeuten,
- R₅, die gleich oder verschieden sind, aus der Reihe Wasserstoffatom, ein geradkettiger oder verzweigter Alkylrest mit 1 bis 12 Kohlenstoffatomen, ein geradkettiger oder verzweigter Alkoxyrest mit 1 bis 6 Kohlenstoffatomen, ein geradkettiger oder verzweigter Acylrest mit 2 bis 30 Kohlenstoffatomen, ein Hydroxylrest, ein Rest -SO₃M, ein gegebenenfalls am Amin substituierter C₁-C₆-Aminoalkoxyrest, ein gegebenenfalls am Amin substituierter C₂-C₆-Aminoacylrest, ein Rest -NHCH₂CH₂COOM, ein Rest -N(CH₂CH₂COOM)₂, ein gegebenenfalls am Amin und an der Alkylkette substituierter Aminoalkylrest, ein C₂-C₃₀-Carboxyacylrest, eine gegebenenfalls durch einen oder zwei substituierte Aminoalkylreste substituierte Gruppe, einen Rest -CO(CH₂)_{d}COOM, einen Rest -COCHR₇(CH₂)_{d}COOM, einen Rest -NHCO(CH₂)_{d}OH, einen Rest -NH₃Y, eine Phosphatgruppe ausgewählt ist,
- M, die gleich oder verschieden sind, ein Wasserstoffatom, Na, K, Li, NH₄ oder ein organisches Amin bedeuten,
- R₇ ein Wasserstoffatom oder einen Rest SO₃M bedeutet,
- d im Bereich von 1 bis 10 liegt,
- m im Bereich von 0 bis 20 liegt,
- n im Bereich von 0 bis 500 liegt,
- o im Bereich von 0 bis 20 liegt,
- p im Bereich von 1 bis 50 liegt,
- a im Bereich von 0 bis 50 liegt,
- b im Bereich von 0 bis 50 liegt,
- a + b größer oder gleich 2 ist,
- c im Bereich von 0 bis 4 liegt,
- x im Bereich von 1 bis 100 liegt,
- Y ein einwertiges anorganisches oder organisches Anion wie Halogenid (Chlorid, Bromid), Sulfat, Carboxylat (Acetat, Lactat, Citrat) bedeutet,
mit der Maßgabe, dass, wenn das Silikon die Formel (XV), wobei R₅ Wasserstoff bedeutet, aufweist, dann n größer als 12 ist,
([Z (R₂SiO)_{q} R'₂SiZO][(CₙH₂ₙO)ᵣ])ₛ (XVIII)
wobei in dieser Formel (XVIII)
- R₂ und R'₂, die gleich oder verschieden sind, einen einwertigen C₁-C₃₀-Kohlenwasserstoffrest bedeuten,
- n eine ganze Zahl von 2 bis 4 bedeutet,
- q eine Zahl gleich oder größer 4, vorzugsweise zwischen 4 und 200 und noch stärker bevorzugt zwischen 4 und 100 bedeutet,
- r eine Zahl gleich oder größer 4, vorzugsweise zwischen 4 und 200 und noch stärker bevorzugt zwischen 5 und 100 bedeutet,
- s eine Zahl gleich oder größer 4, vorzugsweise zwischen 4 und 1 000 und noch stärker bevorzugt zwischen 5 und 300 bedeutet,
- Z eine zweiwertige organische Gruppe, die an das benachbarte Siliciumatom über eine Kohlenstoff-Silicium-Bindung und an den Polyoxyalkylenblock (CₙH₂ₙO) über ein Sauerstoff gebunden ist, bedeutet,
- das mittlere Molekulargewicht von jedem Siloxanblock zwischen ungefähr 400 und ungefähr 10 000 und das von jedem Polyoxyalkylenblock zwischen ungefähr 300 und ungefähr 10 000 liegt,
- die Siloxanblöcke ungefähr 10 Gew.-% bis ungefähr 95 Gew.-% des Blockcopolymers ausmachen,
- das zahlenmittlere Molekulargewicht des Blockcopolymers im Bereich von 2 500 bis 1 000 000 liegen kann.

28. Kosmetische Zusammensetzung nach einem der vorhergehenden Ansprüche, **dadurch gekennzeichnet, dass** sie weiterhin mindestens ein Silikon aus der Reihe der Silikongummis umfasst.

29. Kosmetische Zusammensetzung nach Anspruch 28, **dadurch gekennzeichnet, dass** die Silikongummis aus der Reihe der Polyorganosiloxane mit zahlenmittleren Molekulargewichten zwischen 200 000 und 1 000 000 allein oder in Form einer Mischung in einem Lösungsmittel eingesetzt werden.

30. Kosmetische Zusammensetzung nach einem der Ansprüche 28 oder 29, **dadurch gekennzeichnet, dass** die allein oder in Form einer Mischung eingesetzten Silikongummis aus den folgenden Strukturen:
- Polydimethylsiloxan
- Polydimethylsiloxan/methylvinylsiloxan-Gummis,
- Polydimethylsiloxan/diphenylmethylsiloxan,
- Polydimethylsiloxan/phenylmethylsiloxan,
- Polydimethylsiloxan/diphenylsiloxan/methylvinylsiloxan und
den folgenden Mischungen:
- Mischungen aus einem Polydimethylsiloxan, das am Kettenende hydroxyliert ist, und einem cyclischen Polydimethylsiloxan;
- Mischungen aus einem Polydimethylsiloxangummi und einem cyclischen Silikon;
- Mischungen aus Polydimethylsiloxanen mit unterschiedlichen Viskositäten
ausgewählt sind.

31. Kosmetische Zusammensetzung nach einem der vorhergehenden Ansprüche, **dadurch gekennzeichnet, dass** sie weiterhin mindestens ein zusätzliches fixierendes Polymer umfasst.

32. Kosmetische Zusammensetzung nach einem der vorhergehenden Ansprüche, **dadurch gekennzeichnet, dass** sie weiterhin mindestens ein zusätzliches verdickendes Polymer umfasst.

33. Kosmetische Zusammensetzung nach einem der vorhergehenden Ansprüche, **dadurch gekennzeichnet, dass** sie weiterhin mindestens einen kosmetischen Hilfsstoff, ausgewählt aus der Reihe kationische Tenside, anionische Tenside, amphotere Tenside, nichtionische Tenside, Silikone, bei denen es sich nicht um Silikone der Erfindung handelt, Konditionierungsmittel des Estertyps, Antischaummittel, feuchtigkeitsspendende Mittel, zartmachende Mittel, Weichmacher, silikonhaltige oder nichtsilikonhaltige wasserlösliche und lipidlösliche Sonnenschutzfilter, Färbe- oder Tönungsmittel, Duftstoffe, Peptisierungsmittel, Konservierungsmittel, darunter Ceramide und Pseudoceramide, Vitamine und Provitamine, darunter Panthenol, Proteine, Sequestriermittel, Hilfslösungsmittel, Alkanisierungsmittel, Korrosionsschutzmittel, Fettkörper wie pflanzliche, tierische, mineralische und synthetische Öle, Reduktionsmittel bzw. Antioxidantien, Oxidantien, umfasst.

34. Kosmetische Zusammensetzung nach Anspruch 33, **dadurch gekennzeichnet, dass** der kosmetische Hilfsstoff bzw. die kosmetischen Hilfsstoffe in einer Konzentration von 0,001 bis 50 Gew.-% in Bezug auf das Gesamtgewicht der Zusammensetzung vorliegt/vorliegen.

35. Kosmetische Zusammensetzung nach einem der vorhergehenden Ansprüche, **dadurch gekennzeichnet, dass** es sich bei dem kosmetisch unbedenklichen Medium um ein wässriges, alkoholisches oder wässrig-alkoholisches Medium handelt.

36. Kosmetische Zusammensetzung nach Anspruch 35, **dadurch gekennzeichnet, dass** das wässrig-alkoholische Medium niedrige C₁-C₄-Alkohle, Polyole, Polyolmonoether und ihre Mischungen umfasst.

37. Kosmetische Zusammensetzung nach Anspruch 36, **dadurch gekennzeichnet, dass** es sich bei dem Alkohol um Ethanol handelt.

38. Kosmetische Zusammensetzung nach einem der vorhergehenden Ansprüche, **dadurch gekennzeichnet, dass** sie in Form einer Creme, eines Schaums, einer Lotion, eines Gels, eines Sprays oder eines Mittels nach der Haarwäsche vorliegt.

39. Kosmetische Zusammensetzung nach einem der vorhergehenden Ansprüche, **dadurch gekennzeichnet, dass** sie in einer Sprühflasche, einer Pumpflasche oder einer Aerosoleinheit verpackt ist.

40. Kosmetische Zusammensetzung nach Anspruch 39, **dadurch gekennzeichnet, dass** sie in einer Aerosoleinheit verpackt ist.

41. Kosmetische Zusammensetzung nach Anspruch 40, **dadurch gekennzeichnet, dass** sie ein Treibmittel aus der Reihe Luft, Stickstoff, Kohlendioxid, Dimethylether, C₃- bis C₅-Alkane, 1,1-Difluorethan und ihre Mischungen umfasst.

42. Aerosoleinheit, gebildet aus einem Behältnis, das eine Zusammensetzung nach einem der vorhergehenden Ansprüche sowie ein Mittel zum Verteilen der Zusammensetzung umfasst.

43. Kosmetisches Behandlungsverfahren, **dadurch gekennzeichnet, dass** es die Applikation einer kosmetischen Zusammensetzung nach einem der Ansprüche 1 bis 41, insbesondere auf das Haar, umfasst.

44. Kosmetisches Behandlungsverfahren nach Anspruch 43, **dadurch gekennzeichnet, dass** auf die Applikation der Zusammensetzung kein Spülen erfolgt.

45. Verwendung einer kosmetischen Zusammensetzung nach einem der Ansprüche 1 bis 41 für die Haarpflege.

## Claims

1. Cosmetic composition for treating keratin fibres, in particular human keratin fibres such as the hair, **characterized in that** it comprises, in a cosmetically acceptable medium:
- at least one cationic poly(vinyllactam) polymer comprising:
- a) at least one monomer of vinyllactam or alkylvinyllactam type;
- b) at least one monomer of structure (Ia) or (Ib) below: 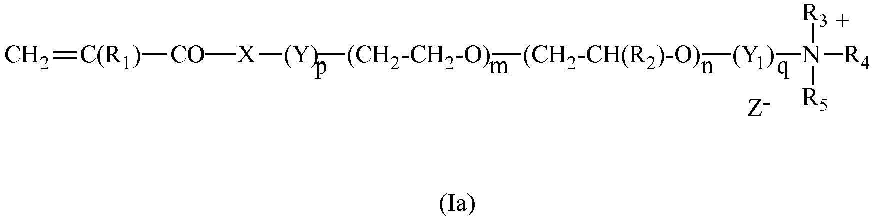 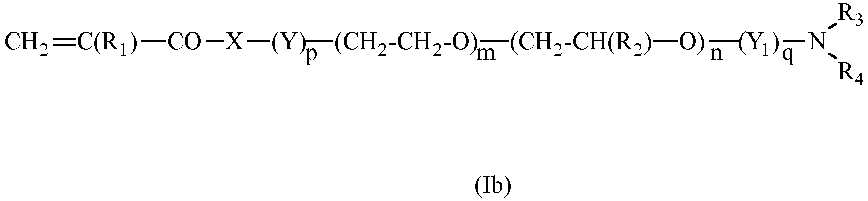 in which:
X denotes an oxygen atom or a radical NR₆,
R₁ and R₆ denote, independently of each other, a hydrogen atom or a linear or branched C₁-C₅ alkyl radical,
R₂ denotes a linear or branched C₁-C₄ alkyl radical,
R₃, R₄ and R₅ denote, independently of each other, a hydrogen atom, a linear or branched C₁-C₃₀ alkyl radical or a radical of formula (II):
- (Y₂)ᵣ-(CH₂-CH(R₇)-O)ₓ-R₈ (II)
Y, Y₁ and Y₂ denote, independently of each other, a linear or branched C₂-C₁₆ alkylene radical,
R₇ denotes a hydrogen atom or a linear or branched C₁-C₄ alkyl radical or a linear or branched C₁-C₄ hydroxyalkyl radical,
R₈ denotes a hydrogen atom or a linear or branched C₁-C₃₀ alkyl radical,
p, q and r denote, independently of each other, either the value 0 or the value 1,
m and n denote, independently of each other, an integer ranging from 0 to 100,
x denotes an integer ranging from 1 to 100,
Z denotes an organic or mineral acid anion,
with the proviso that:
- at least one of the substituents R₃, R₄, R₅ or R₈ denotes a linear or branched C₉-C₃₀ alkyl radical,
- if m or n is other than zero, then q is equal to 1,
- if m or n is equal to zero, then p or q is equal to 0;
- at least one fatty alcohol used in an amount ranging from 0.1% to 30% by weight relative to the total weight of the composition, and
- at least one amino silicone.

2. Cosmetic composition according to Claim 1, **characterized in that** the vinyllactam or alkylvinyllactam monomer is a compound of structure (III) : 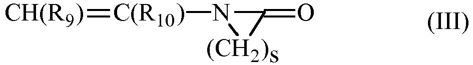 in which:
s denotes an integer ranging from 3 to 6,
R₉ denotes a hydrogen atom or a C₁-C₅ alkyl radical,
R₁₀ denotes a hydrogen atom or a C₁-C₅ alkyl radical,
with the proviso that at least one of the radicals R₉ and R₁₀ denotes a hydrogen atom.

3. Cosmetic composition according to Claim 2, **characterized in that** the monomer of formula (III) is vinylpyrrolidone.

4. Cosmetic composition according to any one of the preceding claims, **characterized in that**, in formula (Ia) or (Ib), the radicals R₃, R₄ and R₅ denote, independently of each other, a hydrogen atom or a linear or branched C₁-C₃₀ alkyl radical.

5. Cosmetic composition according to any one of the preceding claims, **characterized in that** the monomer b) is a monomer of formula (Ia).

6. Cosmetic composition according to Claim 5, **characterized in that**, in formula (Ia), m and n are equal to zero.

7. Cosmetic composition according to any one of the preceding claims, **characterized in that** the counterion Z⁻ of the monomers of formula (Ia) is chosen from halide ions, phosphate ions, the methosulfate ion and the tosylate ion.

8. Cosmetic composition according to any one of Claims 1 to 7, **characterized in that** the cationic poly(vinyllactam) polymer(s) contain(s) one or more additional cationic or nonionic monomers.

9. Cosmetic composition according to Claim 8, **characterized in that** the cationic poly(vinyllactam) is a terpolymer comprising:
a) one monomer of formula (III),
b) one monomer of formula (Ia) in which p=1, q=0, R₃ and R₄ denote, independently of each other, a hydrogen atom or a C₁-C₅ alkyl radical and R₅ denotes a C₉-C₂₄ alkyl radical, and
c) one monomer of formula (Ib) in which R₃ and R₄ denote, independently of each other, a hydrogen atom or a C₁-C₅ alkyl radical.

10. Cosmetic composition according to Claim 9, **characterized in that** the terpolymer comprises, by weight, 40% to 95% of monomer (a), 0.25% to 50% of monomer (b) and 0.1% to 55% of monomer (c).

11. Composition according to any one of the preceding claims, **characterized in that** the cationic poly(vinyllactams) are chosen from vinylpyrrolidone/dimethylaminopropylmethacryl-amide/dodecyldimethylmethacrylamidopropylammonium tosylate terpolymers, vinylpyrrolidone/dimethylamino-propylmethacrylamide/cocoyldimethylmethacrylamido-propylammonium tosylate terpolymers and vinylpyrrolidone/dimethylaminopropylmethacrylamide/lauryldimethyl-methacrylamidopropylammonium tosylate or chloride terpolymers.

12. Composition according to any one of the preceding claims, **characterized in that** the said cationic poly(vinyllactam) polymer is a vinylpyrrolidone/dimethylaminopropyl methacrylamide/lauryldimethyl-methacrylamidopropylammonium chloride terpolymer.

13. Composition according to any one of the preceding claims, **characterized in that** the weight molecular mass of the cationic poly(vinyllactams) is between 500 and 20 000 000, preferably between 200 000 and 2 000 000 and more preferentially between 400 000 and 800 000.

14. Cosmetic composition according to any one of the preceding claims, **characterized in that** the cationic poly(vinyllactams) is (are) used in an amount ranging from 0.05% to 30% by weight, preferably in an amount ranging from 0.1% to 15% by weight and even more preferentially in an amount ranging from 0.2% to 10% by weight relative to the total weight of the composition.

15. Cosmetic composition according to any one of the preceding claims, **characterized in that** the fatty alcohol is linear or branched, and saturated or unsaturated.

16. Cosmetic composition according to Claim 15, **characterized in that** the said non-oxyalkylenated fatty alcohol has the structure R-OH in which R denotes a saturated or unsaturated, linear or branched radical containing from 8 to 40 and preferably from 8 to 30 carbon atoms.

17. Cosmetic composition according to Claim 16, **characterized in that** the said fatty alcohol is chosen from lauryl alcohol, cetyl alcohol, stearyl alcohol, oleyl alcohol, behenyl alcohol, linoleyl alcohol, undecylenyl alcohol, palmitoleyl alcohol, arachidonyl alcohol, erucyl alcohol and cetearyl alcohol, and mixtures thereof.

18. Cosmetic composition according to Claim 17, **characterized in that** the said fatty alcohol is chosen from cetyl alcohol and cetearyl alcohol.

19. Cosmetic composition according to any one of the preceding claims, **characterized in that** the fatty alcohol is solid or pasty at 25°C.

20. Cosmetic composition according to any one of the preceding claims, **characterized in that** the fatty alcohol(s) is (are) used in an amount ranging from 0.2% to 20% by weight and even more preferentially from 0.5% to 10% by weight relative to the total weight of the composition.

21. Cosmetic composition according to any one of the preceding claims, **characterized in that** the amino silicone is chosen from:
(a) the compounds corresponding to formula (V) below:
(R¹)ₐ(T)₃₋ₐ-Si[OSi(T)₂]ₙ-[OSi(T)_{b}(R¹)_{2-b}]ₘ-OSi(T)₃₋ₐ(R¹)ₐ
in which:
T is a hydrogen atom or a phenyl, hydroxyl or C₁-C₈ alkyl radical, and preferably methyl, or a C₁-C₈ alkoxy, preferably methoxy,
a denotes the number 0 or an integer from 1 to 3, and preferably 0,
b denotes 0 or 1, and in particular 1,
m and n are numbers such that the sum (n + m) can range especially from 1 to 2000 and in particular from 50 to 150, n possibly denoting a number from 0 to 1999 and especially from 49 to 149, and m possibly denoting a number from 1 to 2000 and especially from 1 to 10;
R¹ is a monovalent radical of formula -C_{q}H_{2q}L in which q is a number from 2 to 8 and L is an optionally quaternized amino group chosen from the following groups:
- N(R²)-CH₂-CH₂-N(R²)₂
- N(R²)₂
- N^{⊕}(R²)₃Q⁻
- N^{⊕}(R²)(H)₂Q⁻
- N^{⊕}(R²)₂HQ⁻
- N(R²)-CH₂-CH₂-N^{⊕}(R²)(H)₂Q⁻
in which R² can denote a hydrogen atom, a phenyl, a benzyl or a saturated monovalent hydrocarbon-based radical, for example a C₁-C₂₀ alkyl radical, and Q⁻ represents a halide ion such as, for example, fluoride, chloride, bromide or iodide,
(b) the compounds corresponding to formula (VII) below: 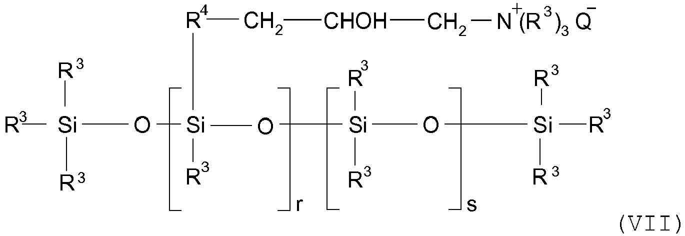 in which
R³ represents a monovalent C₁-C₁₈ hydrocarbon-based radical, and in particular a C₁-C₁₈ alkyl or C₂-C₁₈ alkenyl radical, for example methyl,
R⁴ represents a divalent hydrocarbon-based radical, especially a C₁-C₁₈ alkylene radical or a divalent C₁-C₁₈, and for example C₁-C₈, alkyleneoxy radical;
Q⁻ is a halide ion, especially chloride;
r represents an average statistical value from 2 to 20 and in particular from 2 to 8;
s represents an average statistical value from 20 to 200 and in particular from 20 to 50,
(c) the quaternary ammonium silicones of formula (XII) : 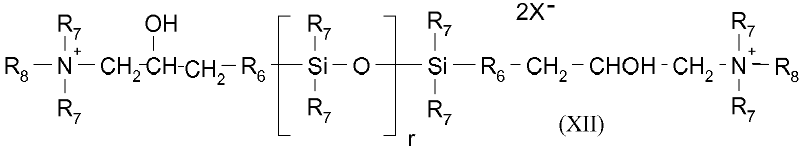 in which:
R₇, which may be identical or different, represent a monovalent hydrocarbon-based radical containing from 1 to 18 carbon atoms, and in particular a C₁-C₁₈ alkyl radical, a C₂-C₁₈ alkenyl radical or a ring containing 5 or 6 carbon atoms;
R₆ represents a divalent hydrocarbon-based radical, especially a C₁-C₁₈ alkylene radical or a divalent C₁-C₁₈ alkyleneoxy radical;
R₈, which may be identical or different, represent a hydrogen atom, a monovalent hydrocarbon-based radical containing from 1 to 18 carbon atoms, and in particular a C₁-C₁₈ alkyl radical, a C₂-C₁₈ alkenyl radical or a radical -R₆-NHCOR₇;
X⁻ is an anion such as a halide ion or an organic acid salt;
r represents a mean statistical value from 2 to 200 and in particular from 5 to 100,
(d) the amino silicones of formula (XIII): 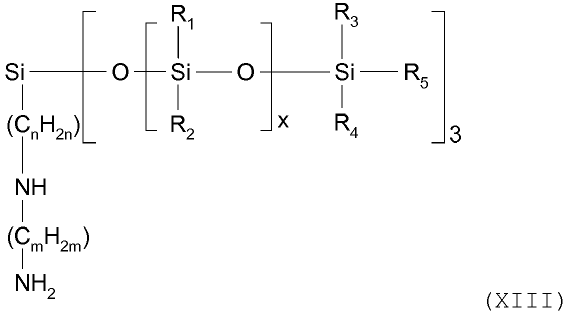 in which:
- R₁, R₂, R₃ and R₄, which may be identical or different, denote a C₁-C₄ alkyl radical or a phenyl group,
- R₅ denotes a C₁-C₄ alkyl radical or a hydroxyl group,
- n is an integer ranging from 1 to 5,
- m is an integer ranging from 1 to 5, and
in which x is chosen such that the amine number is between 0.01 and 1 meq/g.

22. Cosmetic composition according to Claim 21, **characterized in that** the amino silicone corresponding to formula (V) is chosen from compounds corresponding to the following formula: 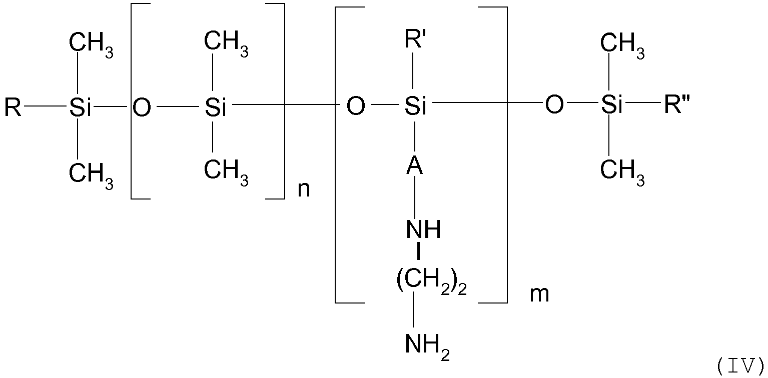 in which R, R' and R", which may be identical or different, denote a C₁-C₄ alkyl radical, preferably CH₃; a C₁-C₄ alkoxy radical, preferably methoxy; or OH; A represents a linear or branched, C₃-C₈ and preferably C₃-C₆ alkylene radical; m and n are integers dependent on the molecular weight and whose sum is between 1 and 2000.

23. Cosmetic composition according to Claim 21, **characterized in that** the amino silicone of formula (V) is the silicone known as "trimethylsilylamodimethicone", corresponding to formula (VI) below: 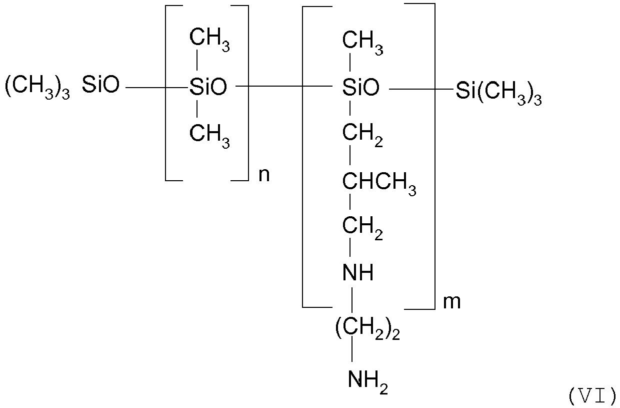 in which n and m have the meanings given in formula (V).

24. Cosmetic composition according to any one of the preceding claims, **characterized in that** the amino silicone(s) is (are) present in an amount ranging from 0.01% to 20% by weight, preferably in an amount ranging from 0.05% to 10% by weight and even more preferentially in an amount ranging from 0.1% to 5% by weight relative to the total weight of the composition.

25. Cosmetic composition according to any one of the preceding claims, **characterized in that** the cationic polyvinyllactam/fatty alcohol weight ratio is between 0.5 and 5, the cationic polyvinyllactam/amino silicone weight ratio is between 0.5 and 5 and the amino silicone/fatty alcohol weight ratio is between 0.5 and 10.

26. Cosmetic composition according to any one of the preceding claims, **characterized in that** it also comprises at least one oxyalkylenated silicone.

27. Cosmetic composition according to Claim 26, **characterized in that** the oxyalkylenated silicone is chosen from the compounds of general formulae (XIV), (XV), (XVI), (XVII) and (XVIII) below: 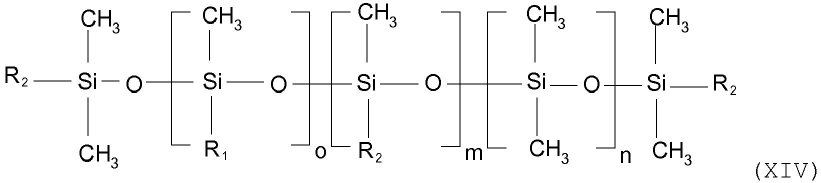 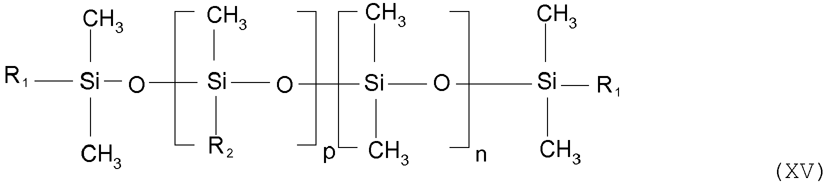 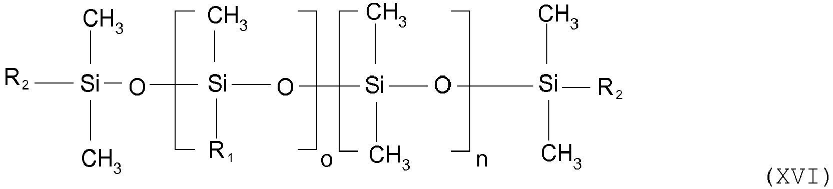 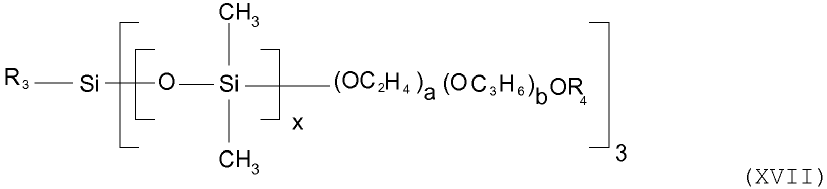 in which:
- R₁, which may be identical or different, represents a linear or branched C₁-C₃₀ alkyl or phenyl radical,
- R₂, which may be identical or different, represents a radical -C_{c}H_{2c}-O- (C₂H₄O)ₐ (C₃H₆O)_{b}-R₅ or a radical -C_{c}H_{2c}-O- (C₄H₈O)ₐ-R₅,
- R₃ and R₄, which may be identical or different, denote a linear or branched C₁ to C₁₂ alkyl radical and preferably a methyl radical,
- R₅, which may be identical or different, is chosen from a hydrogen atom, a linear or branched alkyl radical containing from 1 to 12 carbon atoms, a linear or branched alkoxy radical containing from 1 to 6 carbon atoms, a linear or branched acyl radical containing from 2 to 30 carbon atoms, a hydroxyl radical, -SO₃M, C₁-C₆ aminoalkoxy optionally substituted on the amine, C₂-C₆ aminoacyl optionally substituted on the amine, -NHCH₂CH₂COOM, -N(CH₂CH₂COOM)₂, aminoalkyl optionally substituted on the amine and on the alkyl chain, C₂-C₃₀ carboxyacyl, a group optionally substituted with one or two substituted aminoalkyl radicals, -CO(CH₂)_{d}COOM, -COCHR₇(CH₂)_{d}COOM, -NHCO(CH₂)_{d}OH, -NH₃Y, a phosphate group,
- M, which may be identical or different, denotes a hydrogen atom, Na, K, Li, NH₄ or an organic amine,
- R₇ denotes a hydrogen atom or a radical -SO₃M,
- d ranges from 1 to 10,
- m ranges from 0 to 20,
- n ranges from 0 to 500,
- o ranges from 0 to 20,
- p ranges from 1 to 50,
- a ranges from 0 to 50,
- b ranges from 0 to 50,
- a + b is greater than or equal to 2,
- c ranges from 0 to 4,
- x ranges from 1 to 100,
- Y represents a monovalent mineral or organic anion such as halide (chloride or bromide), sulfate or carboxylate (acetate, lactate or citrate),
with the proviso that when the silicone is of formula (XV) with R₅ denoting hydrogen, then n is greater than 12,
([ Z(R₂SiO)_{q}R'₂SiZO] [(CₙH₂ₙO)ᵣ])ₛ (XVIII)
in which formula (XVIII):
- R₂ and R'₂, which may be identical or different, represent a monovalent C₁-C₃₀ hydrocarbon-based radical,
- n is an integer ranging from 2 to 4,
- q is a number greater than or equal to 4, preferably between 4 and 200 and even more particularly between 4 and 100,
- r is a number greater than or equal to 4, preferably between 4 and 200 and even more particularly between 5 and 100,
- s is a number greater than or equal to 4, preferably between 4 and 1000 and even more particularly between 5 and 300,
- Z represents a divalent organic group linked to the adjacent silicon atom via a carbon-silicon bond and to the polyoxyalkylene block (CₙH₂ₙO) via an oxygen atom,
- the average molecular weight of each siloxane block is between about 400 and about 10 000, the average molecular weight of each polyoxyalkylene block being between about 300 and about 10 000,
- the siloxane blocks represent from about 10% to about 95% by weight of the block copolymer,
- the number-average molecular weight of the block copolymer possibly ranging from 2500 to 1 000 000.

28. Cosmetic composition according to any one of the preceding claims, **characterized in that** it also comprises at least one silicone chosen from silicone gums.

29. Cosmetic composition according to Claim 28, **characterized in that** the silicone gums are chosen from polyorganosiloxanes with number-average molecular masses of between 200 000 and 1 000 000, used alone or in the form of a mixture in a solvent.

30. Cosmetic composition according to either of Claims 28 and 29, **characterized in that** the silicone gums used, alone or in the form of a mixture, are chosen from the following structures:
- polydimethylsiloxane,
- polydimethylsiloxane/methylvinylsiloxane gums,
- polydimethylsiloxane/diphenylsiloxane,
- polydimethylsiloxane/phenylmethylsiloxane,
- polydimethylsiloxane/diphenylsiloxane/methylvinylsiloxane and the following mixtures:
- mixtures formed from a polydimethylsiloxane hydroxylated at the end of the chain and of a cyclic polydimethylsiloxane;
- mixtures formed from a polydimethylsiloxane gum and a cyclic silicone;
- mixtures of polydimethylsiloxanes of different viscosities.

31. Cosmetic composition according to any one of the preceding claims, **characterized in that** it also comprises at least one additional fixing polymer.

32. Cosmetic composition according to any one of the preceding claims, **characterized in that** it also comprises at least one additional fixing polymer.

33. Cosmetic composition according to any one of the preceding claims, **characterized in that** it also comprises at least one cosmetic adjuvant chosen from cationic, anionic, amphoteric or nonionic surfactants, silicones other than those of the invention, conditioning agents of ester type, antifoams, moisturizers, emollients, plasticizers, water-soluble and liposoluble, silicone-based or non-silicone-based sunscreens, permanent or temporary dyes, fragrances, peptizers, preserving agents, ceramides, pseudoceramides, vitamins and provitamins, including panthenol, proteins, sequestrants, solubilizers, basifying agents, anticorrosion agents, fatty substances such as plant, animal, mineral and synthetic oils, reducing agents or antioxidants, and oxidizing agents.

34. Cosmetic composition according to Claim 33, **characterized in that** the cosmetic adjuvant(s) is (are) present in a concentration ranging from 0.001% to 50% by weight relative to the total weight of the composition.

35. Cosmetic composition according to any one of the preceding claims, **characterized in that** the cosmetically acceptable medium is an aqueous, alcoholic or aqueous-alcoholic medium.

36. Cosmetic composition according to Claim 44, **characterized in that** the aqueous-alcoholic medium comprises C₁-C₄ lower alcohols, polyols, polyol monoethers and mixtures thereof.

37. Cosmetic composition according to Claim 36, **characterized in that** the alcohol is ethanol.

38. Cosmetic composition according to any one of the preceding claims, **characterized in that** it is in the form of a cream, a mousse, a lotion, a gel, a spray or a hair conditioner.

39. Cosmetic composition according to any one of the preceding claims, **characterized in that** it is packaged in a vaporiser, a pump-dispenser bottle or an aerosol device.

40. Cosmetic composition according to Claim 39, **characterized in that** it is packaged in an aerosol device.

41. Cosmetic composition according to Claim 40, **characterized in that** it comprises a propellant chosen from air, nitrogen, carbon dioxide, dimethyl ether, C₃-C₅ alkanes and 1,1-difluoroethane, and mixtures thereof.

42. Aerosol device formed by a container comprising a composition according to any one of the preceding claims and a means for distributing the composition.

43. Cosmetic treatment process, **characterized in that** it comprises the application of a cosmetic composition according to any one of Claims 1 to 41, in particular onto the hair.

44. Cosmetic treatment process according to Claim 43, **characterized in that** the application of the said composition is not followed by rinsing.

45. Use of a cosmetic composition according to any one of Claims 1 to 41, for haircare.
